(19) Europäisches Patentamt
European Patent Office
Office européen des brevets

(11) Publication number: **0 451 775 B1**

# (12) EUROPEAN PATENT SPECIFICATION

(45) Date of publication of patent specification: **04.01.95**

(51) Int. Cl.$^{6}$: **C12Q 1/04**, C12Q 1/02, C12Q 1/06, C12Q 1/34, //C12Q1/10

(21) Application number: **91105584.6**

(22) Date of filing: **09.04.91**

## (54) Identifying microorganisms by measuring enzymatic activity in the presence of enzyme activity affecting agents.

(30) Priority: **12.04.90 US 508395**

(43) Date of publication of application:
**16.10.91 Bulletin 91/42**

(45) Publication of the grant of the patent:
**04.01.95 Bulletin 95/01**

(84) Designated Contracting States:
**AT BE CH DE DK ES FR GB GR IT LI LU NL SE**

(56) References cited:
**EP-A- 0 347 771**
**WO-A-86/05206**
**US-A- 4 591 554**

**CHEMICAL ABSTRACTS, vol. 88, no. 5, 30 January 1978, Columbus, OH (US); E.H. PETERSON, p. 230, no. 34280y&NUM;**

**CLINICAL CHEMISTRY, vol. 32, no. 6, June 1986, Winston-Salem, NC (US); C.-C. PAU et al., pp. 987-991&NUM;**

(73) Proprietor: **Becton Dickinson and Company**
**One Becton Drive**
**Franklin Lakes,**
**New Jersey 07417-1880 (US)**

(72) Inventor: **Sussman, Mark L.**
**2104 Smith Avenue**
**Baltimore, MD 21227 (US)**
Inventor: **Berger, Dolores M.**
**1014 Dartmouth Glen Way**
**Baltimore, MD 21212 (US)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

EP 0 451 775 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF CLINICAL MICROBIOLOGY, vol. 21, no. 3, March 1985, Washington, DC (US); S.C. EDBERG et al., pp. 363-365&NUM;

JOURNAL OF CLINICAL MICROBILOGY, vol. 27, no. 8, August 1989, Washington, DC (US); K.J. PANOSIAN et al., pp. 1719-1722&NUM;

## Description

## BACKGROUND OF THE INVENTION

### 1. Field of the invention

This invention relates to a method for identifying microorganisms by measuring the rates of enzymatic hydrolysis of substrates in the presence of enzyme activity affecting agents and comparing these rates to an enzyme activity profile for a variety of identified microorganisms.

### 2. Background of the Related Art

The rapid identification of microorganisms isolated in clinical or laboratory settings is an important goal. Most laboratories identify microorganisms from clinical isolates using commercial bacterial identification systems which require up to 18-24 hours or longer following isolation of the organism to achieve identification. Some of the current "rapid" systems take from 3-13 hours. These systems generally rely upon the detection of the acidic or basic by-products of sugar or amino acid metabolism produced following a period of organism growth. Several of these "growth-based" commercial bacterial identification systems use selected inhibitors to provide information useful for speciating bacteria.

Most notable of these commercial "growth based" systems is the AUTOBAC® IDX manufactured by Organon Teknika, Durham, N.C. This system is used for the identification of gram negative bacilli see, Sielaff et al., "Novel Approach to Bacterial Identification That Uses the Autobac System", Journal of Clinical Microbiology, 15, No. 6, 1103-1110 (1982). Growth inhibition profiles are generated using 18 differential inhibitory chemical agents placed in microbial growth medium, with growth measured nephelo-metrically with the AUTOBAC® IDX instrument. The literature supplied with the AUTOBAC® IDX states that "The profiles are analyzed with a two-stage quadratic discriminant analysis to arrive at the identification." The inhibitors consist of dyes, heavy metal ions, antibiotics, and inhibitors of bacterial metabolism. The test requires a three hour incubation, which generally allows for several generations of bacterial multiplication. The growth in the inhibited wells is compared to the growth in a well containing growth medium but no growth inhibitors.

Bacterial growth is dependent upon many conditions: proper pH and temperature ranges, adequate nutrition to provide energy, and a cascade of enzyme dependent events to build cellular proteins, nucleic acids and other cellular constituents necessary for cell growth and division. The inhibitors used in the AUTOBAC® IDX test can interfere with growth at any of these key points.

Other growth based bacterial identification systems use selective growth inhibitors, but not as pervasively as the AUTOBAC® IDX. Vitek's AUTOMICROBIC SYSTEM®, manufactured by Vitek Systems, St. Louis, Mo. uses Irgasan (DP300) along with a growth medium and an indicator of metabolic activity; see Isenberg et al., "Collaborative Investigation of the AutoMicrobic System Enterobacteriaceae Biochemical Card", Journal of Clinical Microbiology, 11, No. 6, 694-702 (1980).

Cetrimide, another growth inhibitor, is used in the SCEPTOR identification system (Becton Dickinson, Towsen, MD) as a selective inhibitor to discriminate between P. aeruginosa which are not inhibited, and other Psuedomonas species which are inhibited.

All of these growth-based identification systems suffer from the same limitation, they are slow in providing results. Even the so called "rapid" identification systems take from 3-13 hours to obtain their results. The reason why these systems are limited in speed is that all such systems require at least one and often several rounds of microorganism growth and replication.

In many microbial classes there are found enzymes which are common to several or all members of that class. A class being defined as any functionally useful grouping of microorganisms. For example, two functionally useful groupings defining separate classes of microorganisms are the non-lactose fermenting bacteria vs. the lactose fermenting bacteria. Other functionally useful groups are the indole positive vs. the indole negative bacteria. Another grouping which defines classes of microorganisms are the oxidase positive vs. the oxidase negative bacteria. Accordingly, a particular microorganism may be a member of one or several microbial classes. Examples of enzymes which are expressed by members of the genus Enterobacteriaceae are alanine aminopeptidase, alkaline phosphatase, and $\beta$-galactosidase. Esterase enzymes are found among many of the bacteria typically isolated as human pathogens. Various assays, utilizing different substrates and indicator systems have been developed for these common enzyme systems. The very ubiquitous nature of these enzymes limits the utility of prior assays for differentiating among the species making up many of the microbial classes.

One method for identifying specific bacterial species by using the enzymatic cleavage of substrates is described in U.S. Patent No. 4,603,108 to Bascomb. The Bascomb patent describes a kit containing tests for twenty-six (26) constitutive enzymes. In each test, the enzyme is determined by its ability to interact with a specific substrate. A test card or other apparatus has a plurality of wells or compartments which separately contain specific substrate solutions for each of the enzyme tests together with other reagents for the tests. A bacterial suspension is added to each compartment and a detectible product is developed after a relatively short incubation period. The amount of the corresponding enzyme in each sample is then determined by spectrometric analysis using either colorimetry or fluorimetry. In another procedure described in this patent, seven (7) tests are used for rapid differentiation of commonly encountered bacterial groups. Bascomb discloses that either the 26 test assay or the 7 test assay provides a unique fingerprint for the species or group of species. A quantitative determination of enzyme activity for each group of species can be used to identify the group or species by comparison to activity profiles of previously identified bacteria. The specific test described, determines activity by detecting absorbance in a flow cell. Discrete sample analysis and continuous flow analysis can be used. The method of Bascomb, however, requires a large biomass and a high fluid volume, as well as a relatively long incubation time.

Fluorogenic substrates have also been used to assay enzyme activity. These compounds are analogs of naturally occurring metabolites. They typically have a fluorescent moiety bound to the natural metabolite. Enzymatic cleavage of the substrates releases the fluorescent moiety. The fluorescence of the free moiety is much greater than that of the bound moiety. Fluorogenic compounds can thus be used to assay enzyme activity by combining them with the sample in question under appropriate physiological conditions for enzyme activity, and monitoring for an increase in fluorescence.

Researchers have also used fluorogenic substrates to identify microorganisms. Westley et al. in "Aminopeptidase Profiles of Various Bacteria", Appl. Micro., 15, 822-825, (1967) discussed the use of alpha-amino acid β-napthylamide substrates for identification of 24 strains of bacteria. The bacteria were suspended in solution and incubated with substrate solutions. The fluorescence of the released β-naphylamine was measured after 4 hours of incubation. In another publication, Peterson et al., in "Rapid Detection of Selected Gram-Negative bacteria by amino-peptidase profiles"; J. Food Sci. 43 1853-1856 (1978) described the use of fluoro-metric analysis to measure enzyme hydrolysis of nineteen (19) L-amino acid β-napthylamides. A profile for each culture was obtained in 4-6 hours.

A review of the literature pertaining to the use of fluorogenic substrates to profile microbial enzyme activity is contained in Godsey et al., "Rapid Identification of Enterobacteriaceae With Microbial Enzyme Activity Profiles", J. Clin. Micro., 13, 483-490 (1981). The Godsey group reported the use of 18 fluorogenic substrates in a study of 539 strains of the class Enterobacteriaceae. Hydrolysis rates were monitored for the first thirty minutes in 2ml of buffer-containing substrates at 37°C. All substrates except urea were derivatives of β-methylumbelliferone, β-napthylamine or 7-amino-4-methyl coumarin.

In U.S. Patent No. 4,591,554 to Koumara et al., fluorescence analysis using umbelliferone derivatives is described as a method to detect and determine the amount of a small numbers of microorganisms. In this method an umbelliferone derivative was added to a sample solution and then incubated. Thereafter, insoluble residues (e.g. cells) are removed and fluorescence is read using a conventional detector. The level of fluorescence is then related to the number of microorganisms. In some of the examples, co-enzymes are used. In other examples, the cells are disrupted to increase the amount of liberated enzymes.

Fluorogenic substrates are also known to be useful to assay extracellular enzymes present in living organisms. Snyder et al. in "Pattern Recognition Analysis of In-Vivo Enzyme-Substrate Fluorescence Velocities In Micro-organism Detection and Identification", App. & Environ. Micro., 51, 969-997 (1986) reported reaction times of 15 minutes or less. The Snyder group's work also forms the basis of an International Patent Application entitled "Viable Microorganism Detection by Induced Fluorescence" with University of Cincinnati as the applicant, Int'l. Pub. No. WO 86/05206 dated September 12, 1986.

Another technique which is used to fingerprint bacteria is based on the difference in enzyme content and activity as described by Chu-Pong Pal et al., in "A Rapid Enzymatic Procedure for 'Fingerprinting' bacteria by using pattern recognition of 2-dimensional fluorescence data", Clin. Chem., 32, 987-991 (1986). In that system, a mixture of 6 fluorogenic substrates was used, each with a different fluorescent moiety. Fluorescence increases are monitored over a 30-minute period. A Fourier transformation of the fluorescence data is used to produce a two-dimensional array which is characteristic of each test organism.

Edberg et al., in "Measurement of Active Constitutive β-D-Glucosidase (Esculinase) in the Presence of Sodium Deoxycholate", Journal of Clinical Microbiology, Vol. 21. No. 3, 363-365 (1985) disclose a method for differentiating species of the genus Streptococcus by measuring for the enzyme β-D-Glucosidase (Esculinase) with the repression of the enzyme by a bile equivalent (sodium deoxycholate). The method detects the presence of β-D-glucosidase by the hydrolysis of ρ-nitrophenyl-β-D-glucopyranoside, a colormetric substrate. If the substrate is hydrolyzed, ρ-nitrophenyl, a yellow moiety, is released from the colorless parent compound and can be detected by the presence of the yellow color in the sample. In the presence of sodium deoxycholate, most species of Streptococci other than group D are inhibited from releasing β-D-glucosidase (Esculinase). Bile-sensitive Streptococcus pneumoniae can be identified with this system in 30 minutes because of its autolytic induction by sodium deoxycholate.

An improvement of the Edberg et al. method was reported by Panosian and Edberg in "Rapid Identification of Streptococcus bovis by Using Combination Constitutive Enzyme Substrate Hydrolyses", Journal of Clinical Microbiology, V. 27 No. 8, 1719-1722 (Aug. 1989). This procedure utilizes a reagent which contains two hydrolyzable substrates ρ-nitrophenyl-α-D-galactopyranoside (PGAL) and 4-methylumbilliferyl-β-D-glucoside (MGLU) in the presence of sodium deoxycholate. The advantage of this test is that it allows simultaneous detection of 2 constitutive enzymes, α-galactosidase and β-glucosidase in a 30 minute test. This test can identify S. bovis and distinguish Enterococcus spp., viridans group streptococci, S. eguinus, and Streptococcus pneumoniae. The hydrolysis of PGAL is demonstrated by the production of the yellow color and the hydrolysis of MGLU, a fluorogenic substrate, is determined by exposing a tube to a hand-held 4-W 366-nm UV light. Four outcomes from the PGAL-MGLU mixed substrate hydrolysis test are possible, yellow and fluorescent, colorless and fluorescent, yellow and non-fluorescent, and colorless and non-fluorescent.

Although these tests allow rapid differentiation of various species of Streptococcus, the test is very limited, measuring for only two constitutive enzymes. In addition, the test can only yield a positive or negative result for the hydrolysis of substrates. There is no measurements of rates or degrees of hydrolysis, in order to allow differentiation of closely related bacterial classes which produce the same or similar enzymes at different rates.

A device for enhancing fluorescence and kinetics to rapidly identify bacteria and other organisms by their enzymatic hydrolysis of fluorogenic substrates, and methods for using the device are described in the EP-A-0 347 771. The application is assigned to the same assignee as the present invention. The Sussman et al. patent application describes a carrier having at least one kinetics and fluorescence enhancing support mounted on or in it. The dried fluorogenic substrate is dissolved onto the fluorescence enhancing support by first dissolving it in a suitable solvent, and then depositing the solution on the support. The solvent is removed by a drying or vacuum desiccation. Microorganisms derived from biological specimens are identified using fluorogenic substrates, free fluors or both, by adding a fluid sample to one or more of a plurality of the kinetics and fluorescence enhancing supports.

Each of the supports include one of the following fluorogenic substrates or fluors: β-umbelliferone, 7-amino-4-methyl-coumarin, β-napthylamine, fluorescein and resorufin, among others. The enzymes present in the sample hydrolize the substrates. If the substrates are fluorogenic, the hydrolysis rates are determined by measuring the rates of fluorescent product production. If the substrate is not fluorogenic, the kinetics and fluorescence enhancing support has deposited on it an enzyme substrate and a dry free-fluor that is enhanced or quenched in the presence of the hydrolysis product. In this manner, the presence of the enzyme that hydrolizes the particular substrate is detected and if desired, the rate-of-reaction profile of one or more enzymes in the sample is established. The enzyme rate-of-reaction profile is then analyzed and compared with reference enzyme rate-of-reaction profiles of known microorganisms in order to identify the unknown microorganism. Due to strain variation, however, many species of microorganisms can not be sufficiently identified using the device and method of EP-A-0 347 771.

In studying enzyme mechanisms, many compounds can be used to either inhibit or enhance enzyme reaction rates. These compounds have primarily been used as tools to probe the molecular biology of the enzymes and their environs. These compounds include detergents, chelating agents, metal ion co-factors, antibiotics, hydrogen ion concentration and buffering agents, and more specific affectors of enzymatic activity such as competitive inhibitors.

Accordingly, it is an object of the present invention to provide a method for rapidly and accurately identifying microorganisms.

It is a further object of the present invention to advantageously utilize the properties of compounds which affect the enzyme activity of microorganisms in order to provide an extremely rapid and accurate identification system.

## SUMMARY OF THE INVENTION

These and other objects are achieved by the present invention which provides a method for rapidly identifying unidentified microorganisms. According to this method the rates of enzymatic cleavage of a plurality of substrates are determined in the presence of an effective concentration of at least one enzyme cleavage affecting agent which is called an "affector". These substrates are selected from fluorogenic and chromogenic substrates to create a characteristic pattern of enzymatic cleavage rates by enzymes expressed by the unidentified microorganism. This characteristic pattern of enzymatic cleavage rates is a "fingerprint" of the unidentified microorganism when in the presence of the affector. The pattern of enzymatic cleavage rates for the unidentified microorganism is compared to a pattern of enzymatic cleavage rates of the same substrates in the presence of the same affector for one or more identified classes or subclasses of microorganisms. The unidentified microorganism is then identified as belonging to a particular class or subclass of the known microorganisms which most closely matches the pattern of enzymatic cleavage rates in the presence of the affector.

In one embodiment of the present invention, a method for identifying an unidentified microorganism is provided. According to this method, the rate of enzymatic cleavage of a plurality of substrates by at least one enzyme expressed by an unidentified microorganism is determined in the presence of an effective concentration of at least one affector. The substrates can include one or more of the group of fluorogenic or chromogenic substrates, the cleavage of which creates a pattern of enzymatic cleavage rates which are characteristic of the unidentified microorganism in the presence of the affector. The pattern of enzymatic cleavage rates characteristic of the unidentified microorganism are then compared to a pattern of enzymatic cleavage rates, which are characteristic of one or more identified classes or subclasses of microorganisms, for the same substrates obtained in the presence of an affective concentration of the affector. The unknown microorganism is then identified as belonging to the class or subclass of known microorganisms having the pattern of enzymatic cleavage rates of the substrates in the presence of the affector or affectors which most closely correlates to the pattern obtained for the unknown microorganism.

Suitably, the method may further include determining the rate of enzymatic cleavage of one or more of the substrates by at least one enzyme expressed by one or more of the identified classes or subclasses of microorganisms in the presence of an effective concentration of the affector or affectors tested for the unidentified microorganism. In this manner, a pattern of enzymatic cleavage rates in the presence of the affector or affectors is created which is characteristic of each of the identified classes and subclasses of microorganisms. The pattern of enzymatic cleavage rates characteristic of the unidentified microorganism is then compared to the pattern for the cleavage rates, which are characteristic of the identified classes and subclasses of microorganism, for the same substrates obtained in the presence of the affector. The unknown microorganism is then identified as belonging to the class or subclass of known microorganisms having the pattern of enzymatic cleavage rates which most closely correlates to the pattern obtained for the unknown microorganism.

A preferred embodiment of the present invention provides a method for identifying an unidentified microorganism. According to this preferred method, the rates of enzymatic cleavage of a plurality of substrates by at least one enzyme expressed by an unidentified microorganism is determined in the presence of an effective concentration of at least one affector. The substrates can include one or more of the group of fluorogenic or chromogenic substrates, the cleavage of which creates a pattern of enzymatic cleavage rates which are characteristic of the unidentified microorganism in the presence of the affector. The rate of enzymatic cleavage of the same substrates by at least one enzyme expressed by the unidentified microorganism is also determined in the absence of an effective concentration of the affector. In this manner, a pattern of enzymatic cleavage rates which are characteristic of the unidentified microorganism in the absence of the affector is created. A function of the pattern of enzymatic cleavage rates characteristic of the unidentified microorganism in the presence of the affector, and the pattern of enzymatic cleavage rates characteristic of the unidentified microorganism in the absence of the affector is determined. The value of the function determined for the unknown microorganism is compared with a value of a function determined for one or more identified classes of microorganisms. The function for the known microorganisms is determined from a pattern of enzymatic cleavage rates of the substrates obtained in the presence of an effective concentration of the affector and the pattern of enzymatic cleavage rates of the substrates obtained in the absence of the effective concentration of the affector. Accordingly, the unknown microorganism is identified as belonging to the class or subclass of known microorganisms for which the value of the function of the pattern of enzymatic cleavage rates most closely correlates to the value of the function determined from the pattern of enzymatic cleavage rates obtained for the unknown microorganism.

Suitably, the patterns enzymatic cleavage rates for the identified classes of microorganisms can be created according to the present invention both in the presence and in the absence of the affector. Accordingly, the rate of enzymatic cleavage of the substrates by at least one enzyme expressed by one or more identified classes and subclasses of microorganisms is determined in the presence of an effective concentration of at least one affector. In this manner, a pattern of enzymatic cleavage rates characteristic of the identified microorganisms in the presence of the affector is created. Also, the rate of enzymatic cleavage of the same substrates by at least one enzyme expressed by one or more of the identified classes of microorganisms is determined in the absence of an effective concentration of the affector. In this manner, a pattern of enzymatic cleavage rates which are characteristic of the identified microorganisms in the absence of the affector is created. The characteristic patterns of enzymatic cleavage rates for the known classes and subclasses of microorganisms can be used in the method of this invention, as described in the preceding paragraph.

In each of the methods described for the present invention, it is preferred that the enzymatic cleavage rates are determined for two or more classes of microorganisms. Preferably, the substrates cleaved are fluorogenic. These fluorogenic substrates may include a biological analog derivitized with a fluor, such as 7-hydroxy-4-methylcourmarin, 7-amino-4-methylcourmarin, β-napthylamine, fluorescein, resorufin, hydroxypyrenetrisulfonate. The affector used in this method may include sodium dodecyl sulfate, sodium fluoride, sodium chloride, sodium azide, ethylenediamine tetracetic acid, urea, CHAPS, cetylpyridinium chloride, chlorpromazine, 2-phenoxyethanol, cobalt chloride, ouabain, sodium deoxycholate, benzalkonium chloride, cycloserine, ρ-aminosalicylic acid, levamisole or metal ions including Mg, Ca, Zn, Cu, Co, Au, or Hg. Alternatively, they may include a combination or mixture of those affectors. Preferably, the affector can include sodium dodecyl sulfate in a concentration of about 0.5 to about 2.0%. Another preferred affector can include ethylenediaminetetraacetic acid in a concentration of from about 1 to about 100mM. A further preferred affector may include benzalkonium chloride in a concentration of from about 0.01 to about 2%. Another preferred mixture of affectors may include benzalkonium chloride in a concentration of from 0.01 to about 2% and ethylenediaminetetraacetic acid in a concentration of from about 1 to about 100mM.

Particularly preferred substrate and affector combinations are:

4MU-α-D-galactoside
0.1% benzalkonium chloride with 30 mM EDTA
4MU-α-D glucoside
0.1 benzalkonium chloride with 30mM EDTA
4MU-β-D-glucuronide
0.1% benzalkonium chloride with 30mM EDTA
4MU-β-galactoside
1.25% sodium dodecyl sulfate
1.0% benzalkonium chloride
0.75% benzalkonium chloride
0.1 mM chloroauric acid
0.1% benzalkonium chloride with 30 mM EDTA
4MU-β-D-glucoside
30 mM EDTA
4MU-phosphate
10 mM EDTA
5 mM EDTA
10 mM $ZnCl_2$
4MU-nonanoate
0.1% benzalkonium chloride
4MU-palmitate
1% sodium dodecyl sulfate
4MU-stearate
1% sodium dodecyl sulfate
L-arginine AMC
0.1% benzalkonium chloride
L-alanine-AMC
0.75% benzalkonium chloride
2 mM CoCl2
bestatin (0.2 mg/ml)
0.33 mM chloroauric acid
2.5 mM zinc chloride
10 mM EDTA

In many cases, the enzymes expressed by the unidentified microorganism are initially present in the inoculum. Accordingly, for these unidentified microorganisms, the method of this invention for determining the rate of enzymatic cleavage both the presence and in the absence of the affector is very rapid, and the unidentified microorganism can be identified within thirty minutes or less.

For a better understanding of the present invention, reference is made to the following description and examples taken in conjunction with the accompanying tables and figures.

## BRIEF DESCRIPTION OF THE DRAWINGS

Figure 1 is a graph showing the rates of hydrolysis by alanine amino peptidase for four (4) species of Enterobacteriaceae: E. coli, E. cloacae, K. Pneumoniae and C. freundii, as described in Example 1.

Figure 2 is a graph showing the percent enhancement of the hydrolysis rates by alanine amino peptidase for the bacteria described for figure 1, in the presence of 5 mM EDTA, as described in Example 1.

Figure 3 is a graph showing the rates of hydrolysis by β-d galactosidase for three (3) strains each of the four (4) species of Enterobacteriaceae described with regard to Figures 1 and 2, as described in Example 2.

Figure 4 is a graph showing the percent enhancement of β-d galactosidase hydrolysis rates in the presence of one (1%) percent SDS for the same bacterial strains as described in Figure 3, as described in Example 2.

Figure 5 is a graph comparing the rates of hydrolysis of L-alanine 7-amido-4-methyl-coumarin (LAL) both with and without benzalkonium chloride for two strains each of the following bacterial strains: E. coli, E. cloacae, E. aerogenes, K. pneumoniae, C. freundii, P. mirabilis, and M. morganii, as described in Example 3.

Figure 6 is a graph showing the rates of hydrolysis by β-d galactosidase for three (3) strains each of the following microorganisms: K. pneumoniae, E. aerogenes, E. cloacae, C. freundii, and E. coli, as described in Example 4.

Figure 7 is a graph showing the percent of enhancement/suppression of the microorganisms described for Figure 6 of β-d galactosidase rates of hydrolysis in the presence of 1.6% of benzalkonium chloride, as described in Example 4.

Figure 8 is a graph showing the rates of 4-methylumbelliferyl β-d galactoside hydrolysis for three (3) strains each of the same microorganisms described with regard to Figures 7 and 8, as described in Example 5.

Figure 9 is a graph showing the percent enhancement/suppression of β-d galactosidase hydrolysis rates in the presence of 0.8 mM chloroauric acid for three (3) strains each of the same microorganisms described for Figure 8, as described in Example 5.

Figure 10 is a graph showing the rates of enzymatic hydrolysis of L-alanine-7-amido-4-methylcoumarin-(L-alanine -AMC) for three (3) strains each in duplicate of the following microorganisms: E. coli, E. cloacae, E. aerogenes, C. freundii, K. pneumoniae, M. morganii, and P. mirabilis, as described in Example 6.

Figure 11 is a graph showing the rates of hydrolysis of L-alanine-AMC in the presence of 0.8mM cobalt chloride for the same organisms described for Figure 10, as described in Example 6.

Figure 12 is a graph showing the rates of hydrolysis of L-alanine-AMC for the same microorganisms described with regard to Figures 10 and 11, as described in Example 7.

Figure 13 is a graph showing the enhancement/suppression of the rates of hydrolysis of L-alanine-AMC in the presence of 8mM of zinc chloride for the same strains of microorganisms described for Figure 12, as described in Example 7.

Figure 14 is a graph showing the enhancement/suppression of the rates of hydrolysis of 4MU-β-d glucuronide for eight (8) strains of E. Coli, as described in Example 8.

Figure 15 is a graph showing the enhancement/suppression of the rates of hydrolysis of 4MU-β-d glucuronide in the presence of 0.08% benzalkonium chloride and 16 mM EDTA for eight (8) strains of E. Coli, as described in Example 8.

Figures 16A and B are bar graphs representing data ranges for several strains of microorganisms tested on 4MU-phosphate, and 4MU-phosphate with 5mM EDTA, respectvey; while figure 16C is a bar graph of the algorithm values of the data ranges represented in figures 16A and B, as described in Example 9.

Figures 17A, B are bar graphs representing data ranges for several strains of microorganisms tested on 4MU-palmitate, and 4MU-palmitate with 1% SDS, respectively; while figure 17C is a bar graph of the algorithm values of the data ranges represented in figures 17A and B, as described in Example 10.

## Detailed Description of the Invention

Fluorogenic compounds are used in accordance with the present invention to assay enzyme activity. These compounds are analogs of naturally occurring metabolites. They typically have a fluorescent moiety bound to the natural metabolite. Enzymatic cleavage releases this fluorescent moiety. The fluorescence of the free moiety is much greater than that of the bound moiety. These compounds can thus be used to assay enzyme activity by combining them with the sample in question under appropriate physiological conditions for enzyme activity, and monitoring the rate of fluorescence increase.

Examples of fluorogenic cleavage products useful for the present invention are: 7-hydroxy-4-methylcourmarin also designated as β-methylumbelliferone (4MU); 7-amino-4-methylcourmarin (AMC); β-napthylamine; fluorescein; resorufin; hydroxypyrene-trisulfonate. Alternatively, or in combination with fluorogenic cleavage substrates, other indicator cleavage substrates may be utilized in the present invention, including but not limited to chromoenic, radiometric and fluorometric substrates.

In studying enzyme mechanisms, many compounds can be used to either inhibit or enhance enzyme reaction rates. These compounds have primarily been used as tools to probe the molecular biology of the enzymes and their environs. These compounds include detergents, chelating agents, metal ion co-factors, antibiotics, hydrogen ion concentration and buffering agents, and more specific affecters of enzymatic activity such as competitive inhibitors.

By combining fluorogenic substrates with enzymatic cleavage affecting compounds, and using the rates of enzymatic hydrolysis of substrates in the presence of the so called affecter compounds; and by manipulating these rates in combination with other enzyme rate information from non-affected enzymes using the appropriate algorithms, the resulting information can be used to help speciate microbes. For example, a particular fluorogenic substrate may react equally well with two species of bacteria, but the compound may have a more profound effect on the enzymatic cleavage rate with one species of bacteria than another. Examples of such effects can be found in the examples which follow.

A device for enhancing fluorescence and kinetics to rapidly identify bacteria and other organisms by their enzymatic hydrolysis of fluorogenic substrates, and methods for using the device are described in EP-A-0 347 771. The application is assigned to the same assignee as the present invention. The Sussman et al. patent application describes a carrier having at least one kinetics and fluorescence enhancing support mounted on or in it. The dried fluorogenic substrate is dissolved onto the fluorescence enhancing support by first dissolving it in a suitable solvent, and then depositing the solution on the support. The solvent is removed by a drying or vacuum desiccation. Microorganisms derived from biological specimens are identified using fluorogenic substrates, free fluors or both, by adding a fluid sample to one or more of a plurality of the kinetics and fluorescence enhancing supports.

Each of the supports includes a fluorogenic substrate which may release one of the following fluors: 7-hydroxy-4-methylcoumarin, 7-amino-4-methylcoumarin, β-napthylamine, fluorescein and resorufin, among others. The enzymes present in the sample hydrolyze the substrates. If the substrates are fluorogenic, the hydrolysis rates are determined by measuring the rate of fluorescence increase. If the substrate is not fluorogenic, the kinetics and fluorescence enhancing support has deposited on it an enzyme substrate and a dry free-fluor that is enhanced or quenched in the presence of the hydrolysis product. In this manner, the presence of the enzyme that hydrolyses the particular substrate is detected and if desired, the rate-of-reaction profile of one or more enzymes in the sample is established. The enzyme rate-of-reaction profile is then analyzed and compared with reference enzyme rate-of-reaction profiles of known microorganisms in order to identify the unknown microorganism. Due to strain variation and species similarities, however, many species of microorganisms can not be sufficiently identified using the device and method of EP-A-0 347 771.

In the preferred embodiment of the present invention, the device of EP-A-0 347 771 is utilized, but the method is improved by performing the assay in the presence of enzyme activity affecting agents. As illustrated in the following examples, these enzyme activity affecting agents provide a more positive identification for various strains of bacteria. Accordingly, in a preferred embodiment of the present invention, fluorogenic compounds are used to assay enzyme activity.

Examples of useful affector compounds include but are not limited to the following: sodium dodecyl sulfate (SDS); sodium fluoride; sodium chloride; sodium azide; ethylenediamine tetraacetic acid (EDTA); urea; (3-[3-cholamidopropyl)-dimethylamminio]-1-propane sulfonate) (CHAPS); cetylpyridinium chloride; chlorpromazine; 2-phenoxyethanol; cobalt chloride; ouabain; sodium deoxycholate; benzalkonium chloride; cycloserine; p-aminosalicylic acid; levamisole; as well as metal ions of Mg, Ca, Zn, Cu, Co, Au, and Hg. Desired concentrations for these compounds range from 1 uM to 100mM, or preferably from 0.1% to 2% w/v.

Compounds which are particularly preferred are:

SDS 0.5 - 2.0%, pref. 1.0% w/v.

EDTA 1-100mM, pref. 10mM-30mM.

Benzalkonium chloride 0.01-2%, pref. 0.1-0.75% wv.

Benzalkonium chloride/EDTA mixture 0.01-2%, pref.
0.1-0.75% benzalkonium chloride and, 1-100mM, pref.
10mM-30mM EDTA.

Useful substrate and affecter compound combinations are:
4MU-β-D-galactoside - SDS or benzalkonium chloride
4MU-phosphate - EDTA
4MU-palmitate - SDS
4MU-stearate - SDS
L-alanine-AMC - EDTA or benzalkonium chloride

Particularly preferred substrate and affector compound combinations are:
4MU-α-D-galactoside
0.1% benzalkonium chloride with 30 mM EDTA
4MU-α-D glucoside
0.1 benzalkonium chloride with 30mM EDTA
4MU-β-D-glucuronide
0.1% benzalkonium chloride with 30mM EDTA
4MU-β-galactoside
1.25% sodium dodecyl sulfate
1.0% benzalkonium chloride
0.75% benzalkonium chloride
0.1 mM chloroauric acid
0.1% benzalkonium chloride with 30 mM EDTA
4MU-β-D-glucoside
30 mM EDTA
4MU-phosphate
10 mM EDTA
5 mM EDTA
10 mM $ZnCl_2$
4MU-nonanoate
0.1% benzalkonium chloride
4MU-palmitate
1% sodium dodecyl sulfate
4MU-stearate
1% sodium dodecyl sulfate
L-arginine AMC
0.1% benzalkonium chloride
L-alanine-AMC
0.75% benzalkonium chloride
2 mM CoCl2
bestatin (0.2 mg/ml)
0.33 mM chloroauric acid
2.5 mM zinc chloride
10 mM EDTA

Often individual strains of a given species will have rates that tend to cluster for a particular substrate. On this basis one species could be differentiated from another. However, there are also cases in which the individual strains have greatly varied rates, and the rates of many species of organisms overlap. In these cases rates obtained with the substrate alone are compared to rates obtained for substrate with added compound in three ways.

First, rates are directly compared to determine whether strains of each species are more clustered with "affected" rates than with normal rates. This may enable differentiation of one or more species.

Second, a percent inhibition/enhancement is calculated using the formula:

((control rate - "affected" rate) / control rate x 100%).

This formula gives a positive value if the "affected" rate represents inhibition of the control rate, and a negative value if the "affected" rate represents enhancement of the control rate. Often these results may provide differential information where the rates of hydrolysis alone could not. A compound may have an effect on the rates of one species which would be many times greater than the effect it has on another species.

The third method of comparison involves the use of an algorithm which is described in detail in Appendix A. The advantages of using the algorithm are that it provides a noise filter (for variations in small control rates that would produce a percent inhibition/enhancement that has a very large absolute value yet no significance). The algorithm also produces a non-negative value suitable for application in computer programs as described in EP-A-0 347 771 used to analyze the rate data. Various parameters can be adjusted in order to optimize the algorithm. The algorithm considers both the substrate hydrolysis rate with and without the added compound, and uses a function of the ratio of the two rates of hydrolysis to generate a value that is greater than, equal to, or less than a designated neutral value. Values greater than the neutral value represent the enhancement of substrate rates of hydrolysis by affector compounds, values less than the neutral value represent the inhibition of substrate rates of hydrolysis by affector compounds, and values equal to the neutral value represent no significant effect. The logarithm of the algorithm values may also be employed.

The following examples further illustrate the various features of the invention, and are not intended in any way to limit the scope of the invention which is defined in the appended claims.

**EXAMPLE 1**

Alanine aminopeptidase hydrolysis rates of various bacteria were assayed by the following method. 6.25 ug of L-alanine 7-amido-4-methyl-coumarin (Sigma Chemical Co., St. Louis, Mo. 63178) dissolved in ethanol was pipetted onto a 6 mM diameter 740E paper disk (Schleicher and Scheull Inc. Keene, NH 03431) which was adhered to a plastic support. The solvent was removed by evaporation. To the disk 25 uL of a 1 McFarland organism suspension was added. An organism suspension was prepared in a diluent containing in 0.1 M Tris adjusted to pH 8.0, 0.85% NaCl, 0.02% Triton® X-100. Fluorescence was monitored periodically in a Dynatech MicroFLUOR reader (Dynatech Laboratories, Inc., Chantilly, VA 22021). Increase in fluorescence in ten minutes was calculated by subtraction. Relative enzymatic hydrolysis rates for (3) three strains each of four (4) species of Enterobacteriaceae, obtained from clinical isolates, were determined. The rates of hydrolysis by alanine aminopeptidase are presented graphically in Figure 1. Figure 1 shows that the relative rates of substrate hydrolysis by the four (4) species tested overlap, and none of the species can be differentiated from each other using this hydrolysis rate information.

To disks prepared similarly to those described above, ethylenediaminetetraacetic acid (EDTA) (Sigma Chemical Co.) was added to achieve a final concentration after addition of organism suspension of 4 mM EDTA. Rates of substrate hydrolysis were measured. These rates were compared to the control rates for alanine aminopeptidase activity without EDTA. Some species such as Klebsiella pneumoniae showed a moderate decrease in enzymatic activity, while Enterobacter cloacae showed an increase. The hydrolysis rates were compared as percent enhancement and plotted in figure 2 (0% equals no effect due to EDTA addition). Figure 2 shows that the three (3) strains of E. cloacae tested show far more enhancement of alanine aminopeptidase hydrolysis rates due to the addition of 5 mM EDTA than do any of the strains of the other species tested. Thus the feature of percent enhancement is useful for differentiating among these species of Enterobacteriaceae.

**EXAMPLE 2**

Substrate disks containing 25 micrograms each 4-methylumbelliferyl $\beta$-D-galactoside (Sigma Chemical Company) were prepared as described in Example 1. To a like set of paper disks Sodium Dodecyl Sulfate (Sigma Chemical Company) was added at a level of 1%. The final concentration was 0.8% SDS when reconstituted with 25 uL of organism suspension described in Example 1.

Figure 3 shows the change in fluorescence over ten minutes for three strains each of E. coli, E. cloacae, K. pneumoniae and C. freundii. Due to strain variation none of these species could be differentiated by $\beta$-galactosidase rates of hydrolysis. However, when the difference between these rates, and the rates of hydrolysis of the disks containing both 4MU-$\beta$-D-galactoside and SDS was compared, and the results were calculated as percent enhancement, E. coli could be differentiated from the strains of the other three species tested, as shown by the results plotted in figure 4. Thus the effect of SDS on $\beta$-galactosidase rates of hydrolysis becomes the distinguishing feature, rather than the rates of hydrolysis themselves.

**EXAMPLE 3**

As in Example 1, benzalkonium chloride (Sigma Chemical Co.) was added to 6.25 ug of L-alanine 7-amido-4-methyl-coumarin (LAL) to achieve a concentration of 0.4%. Rates of LAL hydrolysis with and without benzalkonium chloride were compared in Figure 5 for two (2) strains each of E. coli, E. cloacae, E. aerogenes, K. pneumoniae, C. freundii, P. mirabilis, and M. morgnii. As shown in figure 5, with the exception of the Proteus and Morganella strains there is considerable data overlap for the alanine aminopeptidase rates, both with and without benzalkonium chloride. Figure 5 also shows that the two (2) strains of Enterobacter aerogenes tested display the greatest enhancement of hydrolysis rates due to the addition of 0.4% benzalkonium chloride. Accordingly, the addition of benzalkonium chloride is useful for differentiating the E. aerogenes species from other members of the Enterobacteriaceae genus.

**EXAMPLE 4**

Substrate disks containing 25 micrograms of 4-methylumbelliferyl β-d galactoside (Sigma Chemical Co., St. Louis, Mo.) were prepared as described in the previous Examples. To a similar set of disks twenty microliters of a 2% Benzalkonium chloride solution was added. The final concentration of Benzalkonium chloride was 1.6% when reconstituted with 25 uL of the organism suspension described in Example 1.

Figure 6 shows the change in fluorescence over ten minutes for three strains each of K. pneumoniae, E. aerogenes, E. cloacae, C. freundii and E. coli. Differentiation of any one species would be difficult on the basis of β-d galactosidase hydrolysis rates. However, after calculating the percentage by which these rates are enhanced in the presence of benzalkonium chloride, differentiation of E. aerogenes from the other four strains became possible, as shown in Fig. 7. Accordingly, in the percentage of β-d galactosidase hydrolysis rate enhancement is useful for differentiating among these various species of Enterobacteriaceae.

**EXAMPLE 5**

Substrate disks containing 25 micrograms each of 4-methylumbelliferyl β-d galactoside were prepared as described in the previous examples. To a similarly prepared set of disks, twenty microliters of 1mM chloroauric acid (AuHC14) was added. The final concentration of chloroauric acid was 0.8mM when reconstituted with 25 microliters of organism suspension as described in Example 1.

Figure 8 shows the change in fluorescence over ten minutes for three strains each of E. coli, E. cloacae, E. aerogenes, C. Freundii and K. Pneumoniae. Differentiation of any one species could not be made from these rates of hydrolysis. However, after calculating the percentage by which these hydrolysis rates are inhibited in the presence of chloroauric acid, as shown in figure 9, differentiation of E. Aerogenes became possible. Accordingly, the percent of inhibition by chloroauric acid of the rate of hydrolysis becomes useful for differentiating among these various species of Enterobacteriaceae.

**EXAMPLE 6**

Paper disks were prepared as described in the previous examples, containing 6.25 micrograms each of L-alanine-7-amido-4-methylcoumarin (L-alanine-AMC). A similar set was prepared which additionally contained cobalt chloride. The final concentration of cobalt chloride was 0.8mM after the addition of 25 microliters of organism suspension described in Example 1.

The change in fluorescence for L-alanine-AMC was measured for several organisms including E. coli, E. cloacae, E. aerogenes, C. freundii, K. pneumoniae, M. morganii and P. mirabilis over a period of ten minutes and plotted in the bar graphs shown in figure 10. As illustrated in Figure 10, no single species could be differentiated on the basis of these rates of hydrolysis alone. These rates of hydrolysis were compared to the rates of hydrolysis when cobalt chloride was added to the L-alanine-AMC. The rates were compared using the algorithm described in Appendix A. This algorithm uses a function of the ratio of the two rates. The results of these comparisons are plotted in Figure 11, showing the values calculated using the algorithm. The values calculated for E. Coli generally fall below the neutral value of zero, while most others do not.

**EXAMPLE 7**

Paper disks were prepared as described in the previous Examples containing 6.25 micrograms each of L-alanine-7-amido-4-methylcourmarin (L-alanine-AMC). A similar set was prepared which also contained zinc chloride. The final concentration of zinc chloride was 8mM after the addition of 25 microliters of organism suspension as described in Example 1.

The change in fluorescence for L-alanine-AMC was measured for several organisms which included, E. coli, E. cloacae, E. aerogenes, C. freundii, K. pneumoniae, M. morganii and P. mirabilis over a period of ten minutes and plotted as the bar graphs shown in figure 12. The results illustrated in figure 12 clearly demonstrate that no single species can be differentiated on the basis of these rates of hydrolysis alone. However, as shown in figure 13, hydrolysis rates for the same substrate with zinc chloride added show a pronounced differentiation of P. mirabilis from all other species tested.

## EXAMPLE 8

Multiple sets (2 per set) of paper disks were prepared as described in Example 1, by adding twenty microliters of a 20mM EDTA solution and drying by evaporation. Next, twenty (20) microliters of a 0.1% benzalkonium chloride solution was added to each of the disks and dried by evaporation. Twenty (20) microliters of a 1.25 mg/ml solution of 4-methylumbelliferyl $\beta$-d glucuronide (BGR) (Sigma Chemical Co., St. Louis, Mo.), in ethanol were then added to both sets of disks, and to another set of similarly prepared disks which did not contain benzalkonium chloride or EDTA. The ethanol was evaporated in a fume hood with a good draft. Upon reconstitution of both types of disks with 25 uL of organism suspension, the final concentration was either 1mg/ml BGR; or, 1mg/ml BGR, 0.08% benzalkonium chloride, and 16mM EDTA.

Figure 14 shows the rates of hydrolysis of 4MU-$\beta$-d glucuronide in nanograms of free fluor per minute for duplicate tests of eight different strains of E. coli. E. coli. is an organism which is considered to be 99% positive for this substrate by conventional (growth based) methods. Note that most strains show very little enzyme activity under these conditions. Figure 15 shows the rates of hydrolysis of 4MU-$\beta$-d glucuronide for the same strains of E. coli when benzalkonium chloride and EDTA are present in the concentrations described above. All eight of the strains show increased enzyme activity in the presence of benzalkonium chloride and EDTA.

## EXAMPLE 9

Paper disks were prepared as described in Example 1, by pipetting 20 microliters per disk of a solution containing 4-methylumbelliferyl phosphate (1.25 mg/ml) and drying by vacuum. Another set of paper disks were pipetted with 20 microliters per disk of a solution containing 4-methylumbelliferyl phosphate (1.25 mg/ml) and ethylenediaminetetraacetic acid (EDTA, 5mM), and dried by vacuum. Upon the addition of 25 microliters of organism suspension, as described in Example 1, the final concentration of EDTA was 4mM.

Five strains each of two species, K. pneumonia and P. Mirabilis, were tested in duplicate. The logarithm of the ratio of the rates of hydrolysis was obtained as described in Example 7 of EP-A-0 347 771. These results are presented as bar graphs in Figure 16. The "L" and "H" represent the low and high data points, respectively, for the distribution of data. The graph is scaled automatically, using the high and low data points as the minimum and maximum values (denoted Min and Max, respectively). "M" denotes the mean value of all the data for that species, "S" to the right of the "M" represents plus one standard deviation, and "S" to the left of the "M" represents minus one standard deviation.

Figure 16A shows that no distinction could be made between K. pneumoniae and P. mirabilis from the rates obtained for 4MU-phosphate alone. Figure 16B clearly shows better separation of the data, such that most of the strains of K. pneumoniae could be separated from most of the strains of P. mirabilis when EDTA is present on the disk with the 4MU-phosphate. Figure 16C shows a bar graph of the algorithm values for the data presented in 16A and 16B, as described in Example 6 and in Appendix A. The algorithm values provide 100% differentiation between K. pneumoniae and P. mirabilis. Hence, the comparison of the rates for 4MU-phosphate alone and with EDTA provides better differentiation than the 4MU-phosphate or 4MU-phosphate with EDTA could provide separately.

## EXAMPLE 10

Paper disks were prepared as described in Example 1, by pipetting 20 microliters per disk of a solution containing 4-methylumbelliferyl palmitate (1.25 mg/ml) and 1.25% sodium dodecyl sulfate (SDS), and also dried by vacuum. Upon the addition of 25 microliters of organism suspension, as described in Example 1, the final concentration of SDS was 1%.

Eleven strains of Mycobacterium gordoneae (MGOR) and 10 strains of M. terrae (MTER) were tested. The logarithm of the ratio of the rates of hydrolysis was obtained as described in Example 7 of commonly assigned U.S. Patent Application Serial No. 209,677. These results are presented as bar graphs in Figure 17. The "L" and "H" represent the low and high data point for the species, respectively. The graph is scaled automatically for the low and high points of the data (denoted as Min and Max, respectively). The "M" represents the mean value for all data of that species, and the "S" values represent the distribution of the data as the mean +/- 1 Standard deviation.

These two closely related species of Mycobacteria are difficult to separate biochemically. while cost members of M. terrae react enzymatically with the 4-methylumbelliferyl palmitate, some members of the species of M. gordoneae also react, causing data overlap in figure 17A. Figure 17B shows greater enzymatic rate enhancement for MGOR than for MTER with the addition of 1% SDS, and these data are shown by the algorithmic expression of this effect in Figure 17C.

Additional experimentation has shown SDS to be useful in affecting the rates of other esterase substrates. Other affector compounds may be used with the esterase substrates as well (i.e. EDTA, benzalkonium chloride, etc.).

## APPENDIX A

The following algorithm compares rates of enzymatic hydrolysis caused by microorganisms in the presence of substrate alone to the rate of enzymatic hydrolysis of the substrate in the presence of an enzyme cleavage affecting compound. The algorithm can be programmed into a computer and used to analyze the ratio of rate data as follows:

Let R = Rate of enzymatic hydrolysis of a substrate in the absence of an enzyme cleavage affecting agent (for example, in units of nanograms of free fluor per minute).

Let I = Rate of enzymatic hydrolysis of a substrate in the presence of an enzyme cleavage affecting compound (for example, in units of nanograms of free fluor per minute).

A portion of the standard deviation of repeated attempts to determine the rates R or I under identical conditions, called K1, is the result of instrument baseline drift, amplifier noise, and like effects, all of which are independent of actual rate. In all likelihood, the significant measured rates have an additional component of their standard deviation, called K2, which is proportional to the square root of the particular rate, R or I. Therefore, the functions R' and I' are defined as the ratio of the particular rate, R or I, respectively, to its expected standard deviation, as follows:

$$R' = X/(K1 + K2 \times \sqrt{X})$$
$$I' = Y/(K1 + K2 \times \sqrt{Y})$$

The following constants are selected in order to optimize the algorithm values and may be adjusted by those of ordinary skill in the art. For the purpose of the present invention, the constants have been selected as follows:

K1 = 0.10
K2 = 0.04
W1 = 1.00
W2 = 1.00, and
W3 = 20.09

If R > 2 x K1, then let X = R
If R ≤ 2 x K1, then let X = 2 x K1
Also, if I > 2 x K1, then let Y = I
If I ≤ 2 x K1, then let Y = 2 x K1

These conditions prevent differences in very small enzymatic rates of hydrolysis (i.e. less than 2 x K1), from appearing significant in the final steps of the algorithm.

The functions F1 through F5 are defined as follows:

```
Let F1 = |I' - R'|  (meaning the absolute value
                     of I' - R')

     then, if F1 ≥ W1, then let F2 = F1.
     If F1 is ≤ or = W1, then let F2 = 0.
     Let  F3 = F2/(F2 + W2)
          F4 = W3 x (1 + F3 x (Y - X)/X)
          F5 = Ln F4 (where Ln is the natural log).
```

The values determined by F1 through F5 have the following significance:

F1 - Tests the magnitude of the difference between the two rates of hydrolysis after compensating for standard deviation. If the magnitude of their differences is one or greater, than the change in rate from R to I is significant.

F2 - Allows a significant rate difference to pass through the algorithm, and filters out insignificant rate differences.

F3 - Has the same function as F2, but makes significant differences appear even more significant by making their value greater than the insignificant values.

F4 - Is the actual algorithm. Note that F4 is a function of the ratio of the corresponding rates of hydrolysis (Y - X)/X.

F5 - Is the natural log (Ln) of F4.

The purpose of the Algorithm is to cluster insignificant rate differences at the neutral value W3, and act as a filter for large differences between very high rates of hydrolysis.

Table 1 demonstrates the algorithm in which hypothetical values have been provided for R and I, as follows:

TABLE 1.

| Organism | R | I | R′ | I′ | F1 | F2 | F3 | F4 | F5 | (I-R)/R |
|---|---|---|---|---|---|---|---|---|---|---|
| 1 | 5.00 | 5.00 | 26.39 | 26.39 | 0.00 | 0.00 | 0.00 | 20.09 | 3.00 | 0.00 |
| 2 | 5.00 | 4.50 | 26.39 | 24.34 | 2.05 | 2.05 | 0.67 | 18.74 | 2.93 | -0.10 |
| 3 | 5.00 | 2.00 | 26.39 | 12.77 | 13.62 | 13.62 | 0.93 | 8.86 | 2.18 | -0.60 |
| 4 | 5.00 | 1.00 | 26.39 | 7.14 | 19.25 | 19.25 | 0.95 | 4.81 | 1.57 | -0.80 |
| 5 | 5.00 | 0.50 | 26.39 | 3.90 | 22.50 | 22.50 | 0.96 | 2.78 | 1.02 | -0.90 |
| 6 | 5.00 | 0.20 | 26.39 | 1.70 | 24.70 | 24.70 | 0.96 | 1.55 | 0.44 | -0.96 |
| 7 | 5.00 | 0.10 | 26.39 | 0.89 | 25.51 | 25.51 | 0.96 | 1.53 | 0.43 | -0.98 |
| 8 | 5.00 | 0.04 | 26.39 | 0.37 | 26.02 | 26.02 | 0.96 | 1.52 | 0.42 | -0.99 |
| 9 | 5.00 | 0.01 | 26.39 | 0.10 | 26.30 | 26.30 | 0.96 | 1.51 | 0.41 | -1.00 |
| 10 | 5.00 | 0.00 | 26.39 | 0.00 | 26.39 | 26.39 | 0.96 | 1.51 | 0.41 | -1.00 |
| 11 | 5.00 | 5.00 | 26.39 | 26.39 | 0.00 | 0.00 | 0.00 | 20.09 | 3.00 | 0.00 |
| 12 | 4.50 | 5.00 | 24.34 | 26.39 | 2.05 | 2.05 | 0.67 | 21.59 | 3.07 | 0.11 |
| 13 | 2.00 | 5.00 | 12.77 | 26.39 | 13.62 | 13.62 | 0.93 | 48.15 | 3.87 | 1.50 |
| 14 | 1.00 | 5.00 | 7.14 | 26.39 | 19.25 | 19.25 | 0.95 | 96.46 | 4.57 | 4.00 |
| 15 | 0.50 | 5.00 | 3.90 | 26.39 | 22.50 | 22.50 | 0.96 | 193.16 | 5.26 | 9.00 |
| 16 | 0.20 | 5.00 | 1.70 | 26.39 | 24.70 | 24.70 | 0.96 | 483.38 | 6.18 | 24.00 |
| 17 | 0.10 | 5.00 | 0.89 | 26.39 | 25.51 | 25.51 | 0.96 | 483.95 | 6.18 | 49.00 |
| 18 | 0.04 | 5.00 | 0.37 | 26.39 | 26.02 | 26.02 | 0.96 | 484.30 | 6.18 | 124.00 |
| 19 | 0.01 | 5.00 | 0.10 | 26.39 | 26.30 | 26.30 | 0.96 | 484.48 | 6.18 | 499.00 |
| 20 | 0.00 | 5.00 | 0.10 | 26.39 | 26.30 | 26.30 | 0.96 | 484.48 | 6.18 | ERR |
| 21 | 0.25 | 5.00 | 2.08 | 26.39 | 24.31 | 24.31 | 0.96 | 386.63 | 5.96 | 19.00 |
| 22 | 0.25 | 4.50 | 2.08 | 24.34 | 22.26 | 22.26 | 0.96 | 346.86 | 5.85 | 17.00 |
| 23 | 0.25 | 2.00 | 2.08 | 12.77 | 10.69 | 10.69 | 0.91 | 148.66 | 5.00 | 7.00 |
| 24 | 0.25 | 1.00 | 2.08 | 7.14 | 5.06 | 5.06 | 0.83 | 70.40 | 4.25 | 3.00 |
| 25 | 0.25 | 0.50 | 2.08 | 3.90 | 1.81 | 1.81 | 0.64 | 33.03 | 3.50 | 1.00 |
| 26 | 0.25 | 0.20 | 2.08 | 1.70 | 0.39 | 0.00 | 0.00 | 20.09 | 3.00 | -0.20 |
| 27 | 0.25 | 0.10 | 2.08 | 0.89 | 1.20 | 1.20 | 0.54 | 17.90 | 2.88 | -0.60 |
| 28 | 0.25 | 0.04 | 2.08 | 0.37 | 1.71 | 1.71 | 0.63 | 17.55 | 2.87 | -0.84 |
| 29 | 0.25 | 0.01 | 2.08 | 0.10 | 1.99 | 1.99 | 0.67 | 17.41 | 2.86 | -0.96 |
| 30 | 0.25 | 0.00 | 2.08 | 0.00 | 2.08 | 2.08 | 0.68 | 17.37 | 2.85 | -1.00 |

TABLE 1 - continued

| Organism | R | I | R' | I' | F1 | F2 | F3 | F4 | F5 | (I-R)/R |
|---|---|---|---|---|---|---|---|---|---|---|
| 31 | 5.00 | 0.25 | 26.39 | 2.08 | 24.31 | 24.31 | 0.96 | 1.76 | 0.56 | -0.95 |
| 32 | 4.50 | 0.25 | 24.34 | 2.08 | 22.26 | 22.26 | 0.96 | 1.93 | 0.66 | -0.94 |
| 33 | 2.00 | 0.25 | 12.77 | 2.08 | 10.69 | 10.69 | 0.91 | 4.01 | 1.39 | -0.88 |
| 34 | 1.00 | 0.25 | 7.14 | 2.08 | 5.06 | 5.06 | 0.83 | 7.51 | 2.02 | -0.75 |
| 35 | 0.50 | 0.25 | 3.90 | 2.08 | 1.81 | 1.81 | 0.64 | 13.61 | 2.61 | -0.50 |
| 36 | 0.20 | 0.25 | 1.70 | 2.08 | 0.39 | 0.00 | 0.00 | 20.09 | 3.00 | 0.25 |
| 37 | 0.10 | 0.25 | 0.89 | 2.08 | 1.20 | 1.20 | 0.54 | 22.82 | 3.13 | 1.50 |
| 38 | 0.04 | 0.25 | 0.37 | 2.08 | 1.71 | 1.71 | 0.63 | 23.26 | 3.15 | 5.25 |
| 39 | 0.01 | 0.25 | 0.10 | 2.08 | 1.99 | 1.99 | 0.67 | 23.43 | 3.15 | 24.00 |
| 40 | 0.00 | 0.25 | 0.00 | 2.08 | 2.08 | 2.08 | 0.68 | 23.48 | 3.16 | ERR |
| 41 | 0.25 | 0.25 | 2.08 | 2.08 | 0.00 | 0.00 | 0.00 | 20.09 | 3.00 | 0.00 |
| 42 | 0.25 | 0.00 | 2.08 | 0.00 | 2.08 | 2.08 | 0.68 | 17.37 | 2.85 | -1.00 |
| 43 | 0.25 | 0.01 | 2.08 | 0.10 | 1.99 | 1.99 | 0.67 | 17.41 | 2.86 | -0.96 |
| 44 | 0.00 | 0.25 | 0.00 | 2.08 | 2.08 | 2.08 | 0.68 | 23.48 | 3.16 | ERR |
| 45 | 0.01 | 0.25 | 0.10 | 2.08 | 1.99 | 1.99 | 0.67 | 23.43 | 3.15 | 24.00 |
| 46 | 0.25 | -0.25 | 2.08 | 0.00 | 2.08 | 2.08 | 0.68 | 17.37 | 2.85 | -2.00 |
| 47 | 10.00 | -0.20 | 44.15 | 0.00 | 44.15 | 44.15 | 0.98 | 0.84 | 0.00 | -1.02 |
| 48 | 1E+2 | 0.25 | 2E+2 | 2.08 | 2E+2 | 2E+2 | 0.99 | 0.15 | 0.00 | -1.00 |
| 49 | 1E+2 | -2.00 | 2E+2 | 0.00 | 2E+2 | 2E+2 | 1.00 | 0.14 | 0.00 | -1.02 |

## Claims

**1.** A method for identifying an unidentified microorganism, comprising:

determining the rate of enzymatic cleavage of a plurality of substrates by at least one enzyme expressed by an unidentified microorganism in the presence of an effective concentration of at least one enzyme cleavage affecting agent (affector), said substrates selected from the group consisting of fluorogenic and chromogenic substrates to create a pattern of enzymatic cleavage rates which are characteristic of the unidentified microorganism in the presence of said affecting agent; and

identifying said unknown microorganism as belonging to the class of known microorganisms for which the pattern of enzymatic cleavage rates of the substrates in the presence of said affector most closely correlates to a pattern obtained for the unknown microorganism.

**2.** The method for identifying an unidentified microorganism as recited in Claim 1, further comprising comparing the pattern of enzymatic cleavage rates characteristic of said unidentified microorganism with a pattern of enzymatic cleavage rates of said substrates obtained in the presence of an effective concentration of said affecting agent for one or more identified classes of microorganism.

**3.** The method for identifying an unidentified microorganism as recited in Claim 1, wherein said substrates comprise fluorogenic substrates.

**4.** A method for identifying an unidentified microorganism, comprising:

determining the rate of enzymatic cleavage of a plurality of substrates by at least one enzyme expressed by an unidentified microorganism in the presence of an effective concentration of at least one enzyme cleavage affecting agent (affector), said substrates selected from the group consisting of fluorogenic and chromogenic substrates to create a pattern of enzymatic cleavage rates which are characteristic of the unidentified microorganism in the presence of said affecting agent;

determining the rate of enzymatic cleavage of said substrates by at least one enzyme expressed by one or more identified classes or subclasses of microorganisms in the presence of an effective concentration at least one affector to create a pattern of enzymatic cleavage rates characteristic of said identified microorganisms in the presence of said affector;

comparing the pattern of enzymatic cleavage rates characteristic of said unidentified microorganism with said pattern of enzymatic cleavage rates of the substrates in the presence of the affector which most closely correlates to the pattern obtained for the unknown microorganism.

**5.** The method for identifying an unidentified microorganism as recited in Claim 4, wherein said substrates comprise fluorogenic substrates.

**6.** A method for identifying an unidentified microorganism, comprising:

determining the rate of enzymatic cleavage of a plurality of substrates by at least one enzyme expressed by an unidentified microorganism in the presence of an effective concentration of at least one enzyme cleavage affecting agent (affector), said substrates selected from the group consisting of fluorogenic and chromogenic substrates, to create a pattern of enzymatic cleavage rates which are characteristic of the unidentified microorganism in the presence of said affecting agent;

determining the rate of enzymatic cleavage of said substrates by at least one enzyme expressed by one or more of said unidentified microorganism in the absence of an effective concentration of said affector to create a pattern of enzymatic cleavage rates which are characteristic of said unidentified microorganism in the absence of said affector;

comparing a function of the pattern of enzymatic cleavage rates characteristic of said unidentified microorganism in the presence of said affector to the pattern of enzymatic cleavage rates characteristic of said unidentified microorganism in the absence of said affector, with a function of a ratio of a pattern of enzymatic cleavage rates of said substrates obtained in the presence of an effective concentration of said affector for one or more identified classes or subclasses of microorganisms; and,

identifying said unknown microorganism as belonging to the class or subclass of known microorganisms for which the function of the pattern of enzymatic cleavage rates most closely correlates to the function of the pattern of enzymatic cleavage rates obtained for the unknown microorganism.

**7.** The method for identifying an unidentified microorganism as recited in Claim 6, wherein said patterns of enzymatic cleavage rates for said identified classes of microorganisms are created by

determining the rate of enzymatic cleavage of said substrates by at least one enzyme expressed by one or more identified class or subclasses of microorganisms in the presence of an effective concentration of said at least one affector to create a pattern of enzymatic cleavage rates characteristic of said identified microorganism in the presence of said affector, and

determining the rate of enzymatic cleavage of said substrates by at least one enzyme expressed by one or more of said identified classes or subclasses of microorganism in the absence of an effective concentration of said affector to create a pattern of enzymatic cleavage rates characteristic of said identified microorganism in the absence of said affector.

**8.** The method for identifying an unidentified microorganism as recited in Claim 7, wherein said substrate comprises a fluorogenic substrate.

**9.** The method for identifying an unidentified microorganism as recited in Claim 7, further comprising identifying said unknown microorganism as belonging to a known subclass of said class of microorganism for which the pattern of enzymatic cleavage rates of the substrates in the presence of said affector most closely correlates to the pattern obtained for the unknown microorganism.

**Patentansprüche**

**1.** Verfahren zum Identifizieren eines nicht identifizierten Mikroorganismus, umfassend:
Bestimmen der Geschwindigkeit der enzymatischen Spaltung einer Vielzahl von Substraten durch wenigstens ein von einem nicht identifizierten Mikroorganismus exprimiertes Enzym in Gegenwart einer wirksamen Konzentration wenigstens eines die Enzymspaltung beeinflussenden Mittels (Affektor), wobei die Substrate aus der aus fluorogenen und chromogenen Substraten bestehenden Gruppe ausgewählt sind, wodurch ein Muster enzymatischer Spaltungs-Geschwindigkeiten erzeugt wird, die für die nicht identifizierten Mikroorganismen in Gegenwart des beeinflussenden Mittels charakteristisch sind; und
Identifizieren der nicht identifizierten Mikroorganismen als zu der Klasse der identifizierten Mikroorganismen gehörend, für die das Muster der enzymatischen Spaltungs-Geschwindigkeiten der Substrate in Gegenwart des Affektors am stärksten mit einem Muster korreliert, das für den nicht identifizierten Mikroorganismus erhalten wurde.

**2.** Verfahren zum Identifizieren eines nicht identifizierten Mikroorganismus nach Anspruch 1, weiterhin umfassend das Vergleichen des Musters der für den nicht identifizierten Mikroorganismus charakteristischen enzymatischen Spaltungs-Geschwindigkeiten mit einem Muster enzymatischer Spaltungs-Geschwindigkeiten der Substrate, das in Gegenwart einer effektiven Konzentration des beeinflussenden Mittels für eine oder mehrere identifizierte Klassen von Mikroorganismen erhalten wurde.

**3.** Verfahren zum Identifizieren eines nicht identifizierten Mikroorganismus nach Anspruch 1, wobei das Substrat fluorogene Substrate umfaßt.

**4.** Verfahren zum Identifizieren eines nicht identifizierten Mikroorganismus, umfassend:
Bestimmen der Geschwindigkeit der enzymatischen Spaltung einer Vielzahl von Substraten durch wenigstens ein von einem nicht identifizierten Mikroorganismus exprimiertes Enzym in Gegenwart einer wirksamen Konzentration wenigstens eines die Enzymspaltung beeinflussenden Mittels (Affektor), wobei die Substrate aus der aus fluorogenen und chromogenen Substraten bestehenden Gruppe ausgewählt sind, wodurch ein Muster enzymatischer Spaltungs-Geschwindigkeiten erzeugt wird, die für die nicht identifizierten Mikroorganismen in Gegenwart des beeinflussenden Mittels charakteristisch sind;
Bestimmen der Geschwindigkeit der enzymatischen Spaltung der Substrate durch wenigstens ein von einer oder mehrerer identifizierter Klassen oder Unterklassen von Mikroorganismen exprimiertes Enzym in Gegenwart einer wirksamen Konzentration wenigstens eines Affektors, wodurch ein Muster enzymatischer Spaltungs-Geschwindigkeiten der identifizierten Mikroorganismen in Gegenwart des Affektors erzeugt wird;
Vergleichen des Musters der für den nicht identifizierten Mikroorganismus typischen enzymatischen Spaltungs-Geschwindigkeiten mit den Mustern der enzymatischen Spaltungs-Geschwindigkeiten der Substrate in Gegenwart des Affektors, die am stärksten mit dem für den unbekannten Mikroorganismus erhaltenen Muster korrelieren.

**5.** Verfahren zum Identifizieren eines unbekannten Mikroorganismus nach Anspruch 4, wobei die Substrate fluorogene Substrate umfassen.

**6.** Verfahren zum Identifizieren eines unbekannten Mikroorganismus, umfassend:
Bestimmen der Geschwindigkeit der enzymatischen Spaltung einer Vielzahl von Substraten durch wenigstens ein von einem nicht identifizierten Mikroorganismus exprimiertes Enzym in Gegenwart einer wirksamen Konzentration wenigstens eines die enzymatischen Spaltung beeinflussenden Mittels (Affektor), wobei das Substrat aus der aus fluorogenen und chromogenen Substraten bestehenden Gruppe ausgewählt ist, wodurch ein Muster enzymatischer Spaltungs-Geschwindigkeiten gebildet wird, die charakteristisch für die nicht identifizierten Mikroorganismen in Anwesenheit des beeinflussenden Mittels sind;
Bestimmen der Geschwindigkeit der enzymatischen Spaltung der Substrate durch wenigstens ein von einem oder mehreren der nicht identifizierten Mikroorganismen exprimiertes Enzym in Abwesenheit einer wirksamen Konzentration des Affektors, wodurch ein Muster enzymatischer Spaltungs-Geschwindigkeiten erzeugt wird, die für die nicht identifizierten Mikroorganismen in Abwesenheit des beeinflussenden Mittels charakteristisch sind;
Vergleichen einer Funktion des Musters der für den nicht identifizierten Mikroorganismus in Gegenwart des Affektors charakteristischen enzymatischen Spaltungs-Geschwindigkeiten mit dem Muster der für den nicht identifizierten Mikroorganismus in Abwesenheit des Affektors für den Mikroorganismus charakteristischen enzymatischen Spaltungs-Geschwindigkeiten mit einer Funktion eines Verhältnisses eines Musters enzymatischer Spaltungs-Geschwindigkeiten der Substrate, erhalten in Gegenwart einer effektiven Konzentration des Affektors für eines oder mehrere identifizierte Klassen oder Unterklassen von Mikroorganismen; und
Identifizieren des unbekannten Mikroorganismus als zu der Klasse oder Unterklasse von bekannten Mikroorganismen gehörend, für die die Funktion des Musters der enzymatischen Spaltungs-Geschwindigkeiten am engsten mit der Funktion des Musters der enzymatischen Spaltungs-Geschwindigkeiten, wie sie für den unbekannten Mikroorganismus erhalten wurde, korreliert.

**7.** Verfahren zum Identifizieren eines nicht identifizierten Mikroorganismus nach Verfahren 6, wobei die Muster der enzymatischen Spaltungs-Geschwindigkeiten für die identifizierten Klassen der Mikroorganismen erzeugt werden durch:
Bestimmen der Geschwindigkeit der enzymatischen Spaltung der Substrate durch wenigstens ein von einer oder mehreren identifizierten Klassen oder Unterklassen von Mikroorganismen exprimiertes Enzym in Gegenwart einer wirksamen Konzentration des wenigstens einen Affektors, wodurch ein Muster enzymatischer Spaltungs-Geschwindigkeiten erzeugt wird, das für die identifizierten Mikroorganismen in Gegenwart des Affektors charakteristisch ist, und
Bestimmen der Geschwindigkeit der enzymatischen Spaltung der Substrate durch wenigstens ein von einer oder mehreren der identifizierten Klassen oder Unterklassen von Mikroorganismen exprimiertes Enzym in Abwesenheit einer wirksamen Konzentration des Affektors, wodurch ein Muster enzymatischer Spaltungs-Geschwindigkeiten erzeugt wird, das für die identifizierten Mikroorganismen in Abwesenheit des Affektors charakteristisch ist.

**8.** Verfahren zum Identifizieren eines nicht identifizierten Mikroorganismus nach Anspruch 7, wobei das Substrat ein fluorogenes Substrat umfaßt.

**9.** Verfahren zum Identifizieren eines nicht identifizierten Mikroorganismus nach Anspruch 7, weiterhin umfassend das Identifizieren des unbekannten Mikroorganismus als zu einer bekannten Unterklasse der Klasse von Mikroorganismen gehörend, für die das Muster der enzymatischen Spaltungs-Geschwindigkeiten der Substrate in Gegenwart des Affektors am stärksten mit dem für den unbekannten Mikroorganismus erhaltenen Muster korreliert.

**Revendications**

**1.** Procédé d'identification d'un microorganisme non identifié, comprenant:

la détermination de la vitesse de coupure enzymatique de plusieurs substrats par au moins une enzyme exprimée par un microorganisme non identifié en présence d'une concentration efficace d'au moins un agent affectant la coupure enzymatique (affecteur), lesdits substrats étant choisis dans le groupe constitué par les substrats fluorogènes et les substrats chromogènes, pour créer un profil de vitesses de coupure enzymatique qui soient caractéristiques du microorganisme non identifié en présence dudit agent affecteur; et

l'identification dudit microorganisme inconnu comme appartenant à la classe de microorganismes connus pour lesquels le profil de vitesses de coupure enzymatique des substrats en présence dudit affecteur est le plus proche d'un profil obtenu à partir du microorganisme inconnu.

2. Procédé d'identification d'un microorganisme non identifié tel que décrit dans la revendication 1, comprenant aussi la comparaison du profil de vitesses de coupure enzymatique caractéristique dudit microorganisme non identifié avec un profil de vitesses de coupure enzymatique desdits substrats obtenus en présence d'une concentration efficace dudit agent affecteur pour une ou plusieurs classes identifiées de microorganismes.

3. Procédé d'identification d'un microorganisme non identifié tel que décrit dans la revendication 1, dans lequel lesdits substrats comprennent des substrats fluorogènes.

4. Procédé d'identification d'un microorganisme non identifié comprenant:
la détermination de la vitesse de coupure enzymatique de plusieurs substrats par au moins une enzyme exprimée par un microorganisme non identifié en présence d'une concentration efficace d'au moins un agent affectant la coupure enzymatique (affecteur), lesdits substrats étant choisis dans le groupe constitué par les substrats fluorogènes et les substrats chromogènes, pour créer un profil de vitesses de coupure enzymatique qui soient caractéristiques du microorganisme non identifié en présence dudit agent affecteur;
la détermination de la vitesse de coupure enzymatique desdits substrats par au moins une enzyme exprimée par une ou plusieurs classes ou sous-classes identifiées de microorganismes en présence d'une concentration efficace d'au moins un affecteur, pour créer un profil de vitesses de coupure enzymatique caractéristique desdits microorganismes identifiés en présence dudit agent affecteur;
la comparaison du profil de vitesses de coupure enzymatique caractéristique dudit microorganisme non identifié avec ledit profil de vitesses de coupure enzymatique des substrats en présence de l'affecteur (agent affectant la coupure enzymatique) le plus proche du profil obtenu pour le microorganisme inconnu.

5. Procédé d'identification d'un microorganisme non identifié tel que décrit dans la revendication 4, dans lequel lesdits substrats comprennent des substrats fluorogènes.

6. Procédé d'identification d'un microorganisme non identifié comprenant:
la détermination de la vitesse de coupure enzymatique de plusieurs substrats par au moins une enzyme exprimée par un microorganisme non identifié en présence d'une concentration efficace d'au moins un agent affectant la coupure enzymatique (affecteur), lesdits substrats étant choisis dans le groupe constitué par les substrats fluorogènes et les substrats chromogènes, pour créer un profil de vitesses de coupure enzymatique qui soient caractéristiques du microorganisme non identifié en présence dudit agent affecteur;
la détermination de la vitesse de coupure enzymatique desdits substrats par au moins une enzyme exprimée par un ou plusieurs desdits microorganismes non identifiés en l'absence d'une concentration efficace dudit affecteur, pour créer un profil de vitesses de coupure enzymatique qui soient caractéristiques dudit microorganisme non identifié en l'absence dudit affecteur;
la comparaison de la fonction donnant le profil de vitesses de coupure enzymatique caractéristique dudit microorganisme non identifié en présence dudit affecteur par rapport au profil de vitesses de coupure enzymatique caractéristique dudit microorganisme non identifié en l'absence dudit affecteur, avec une fonction de la fraction d'un profil de vitesses de coupure enzymatique desdits substrats obtenu en présence d'une concentration efficace dudit affecteur pour une ou plusieurs classes ou sous-classes identifiées de microorganismes; et
l'identification dudit microorganisme inconnu comme appartenant à la classe ou à la sous-classe de microorganismes connus pour lesquels la fonction du profil de vitesses de coupure enzymatique est la plus proche de la fonction du profil de vitesses de coupure enzymatique obtenue pour le microorganisme inconnu.

7. Procédé d'identification d'un microorganisme non identifié tel que décrit dans la revendication 6, dans lequel lesdits profils de vitesses de coupure enzymatique pour lesdites classes identifiées de microorganismes sont créés par
détermination de la vitesse de coupure enzymatique desdits substrats par au moins une enzyme exprimée par une ou plusieurs classes ou sous-classes identifiées de microorganismes en présence d'une concentration efficace dudit affecteur en nombre d'au moins un pour créer un profil de vitesses de coupure enzymatique caractéristique dudit microorganisme identifié en présence dudit affecteur, et
détermination de la vitesse de coupure enzymatique desdits substrats par au moins une enzyme exprimée par une ou plusieurs desdites classes ou sous-classes identifiées de microorganismes en l'absence d'une concentration efficace dudit affecteur pour créer un profil de vitesses de coupure enzymatique caractéristique dudit microorganisme identifié en l'absence dudit affecteur.

**8.** Procédé d'identification d'un microorganisme non identifié tel que décrit dans la revendication 7, dans lequel ledit substrat comprend un substrat fluorogène.

**9.** Procédé d'identification d'un microorganisme non identifié tel que décrit dans la revendication 7, comprenant aussi l'identification dudit microorganisme inconnu comme appartenant à une sous-classe connue de ladite classe de microorganismes pour laquelle le profil de vitesses de coupure enzymatique des substrats en présence dudit affecteur est le plus proche du profil obtenu pour le microorganisme inconnu.

FIG.1

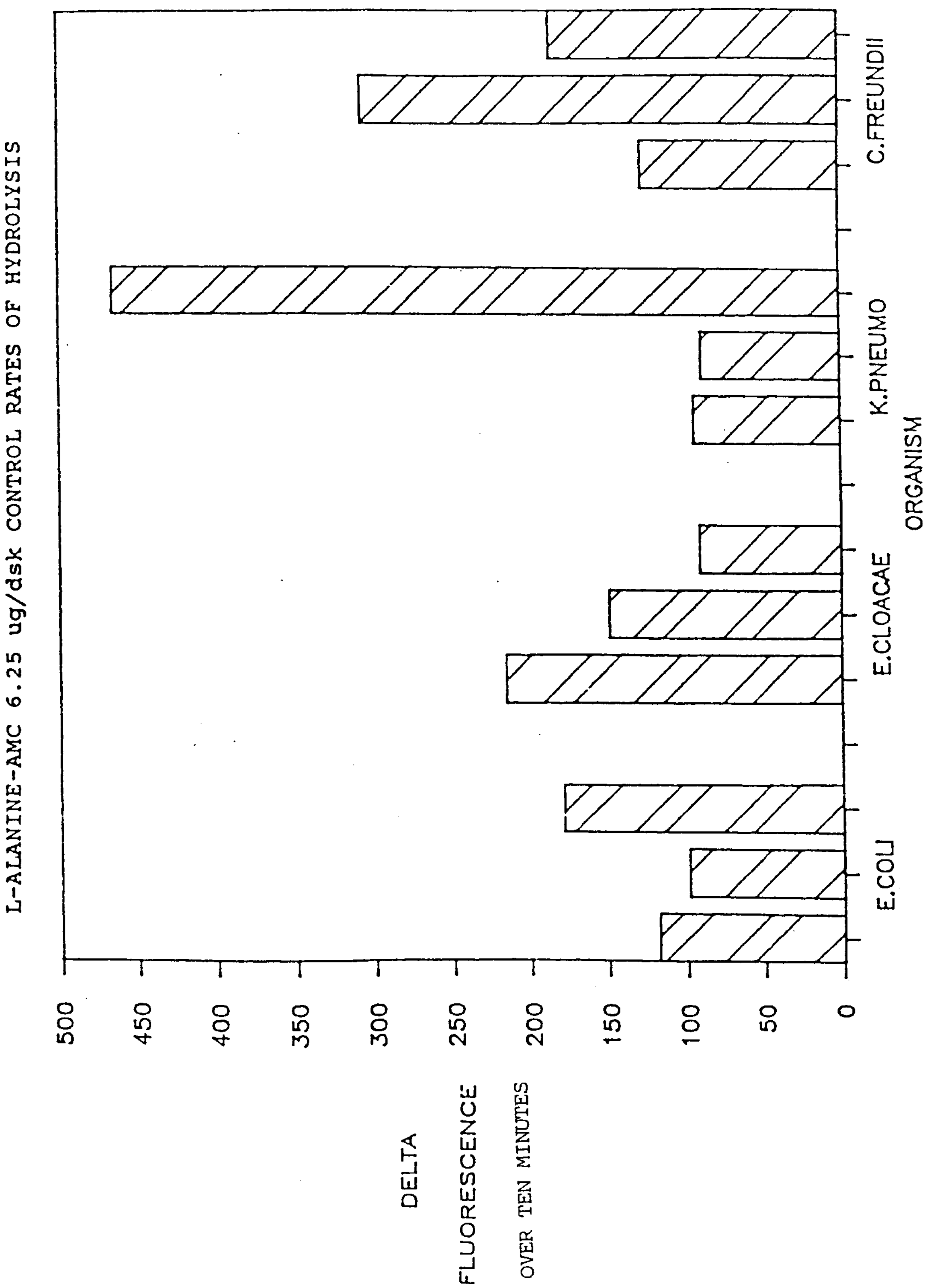
L-ALANINE-AMC 6.25 ug/dsk CONTROL RATES OF HYDROLYSIS
DELTA
FLUORESCENCE
OVER TEN MINUTES
500
450
400
350
300
250
200
150
100
50
0
E.COLI
E.CLOACAE
K.PNEUMO
C.FREUNDII
ORGANISM

FIG.2

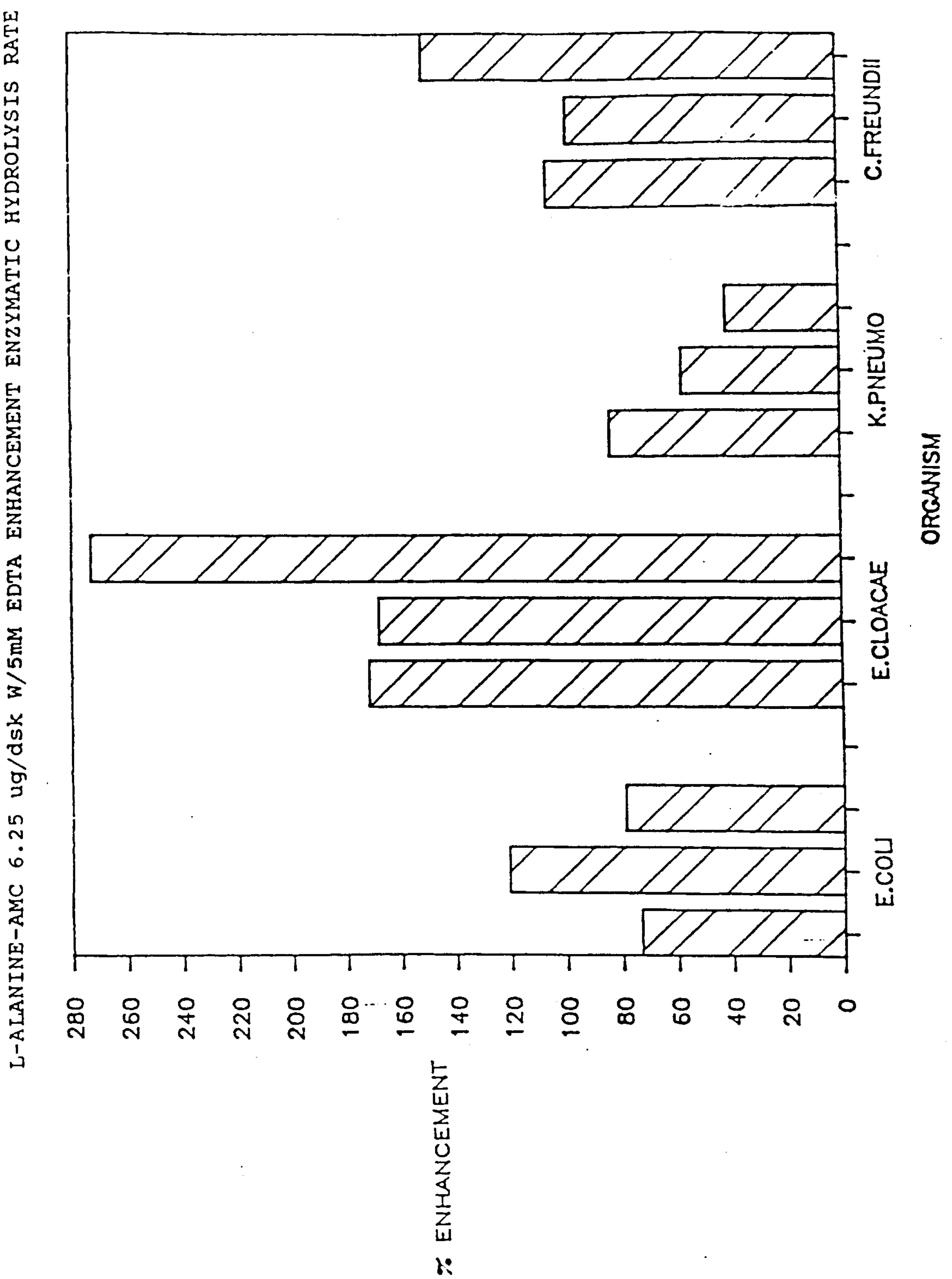
L-ALANINE-AMC 6.25 ug/dsk W/5mM EDTA ENHANCEMENT ENZYMATIC HYDROLYSIS RATE
% ENHANCEMENT
280
260
240
220
200
180
160
140
120
100
80
60
40
20
0
E.COLI
E.CLOACAE
K.PNEUMO
C.FREUNDII
ORGANISM

FIG.3

4MU-B-D GALACTOSIDE 25 ug/disk, CONTROL RATES OF HYDROLYSIS

Three Strains Each Species

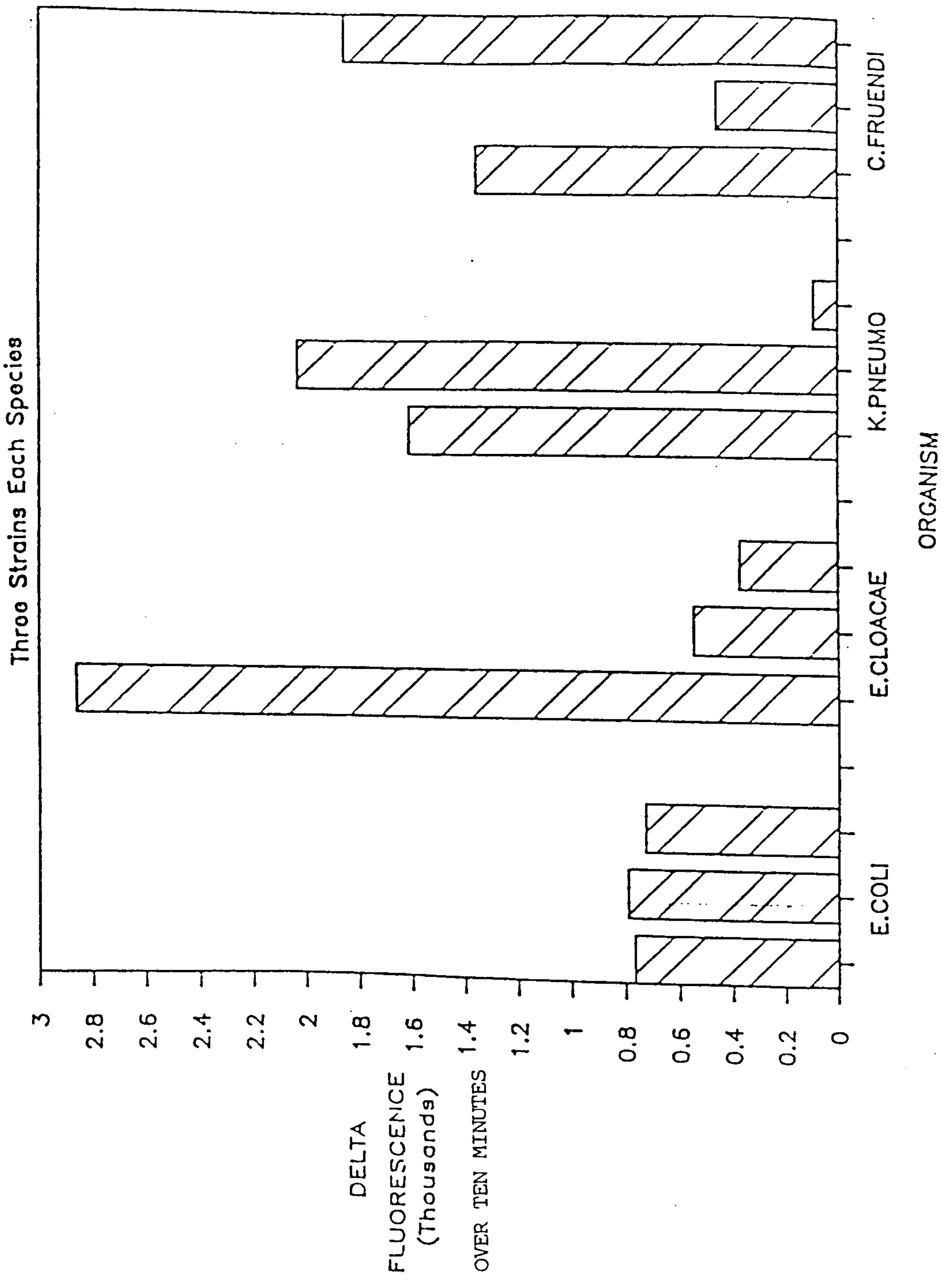

FIG. 4

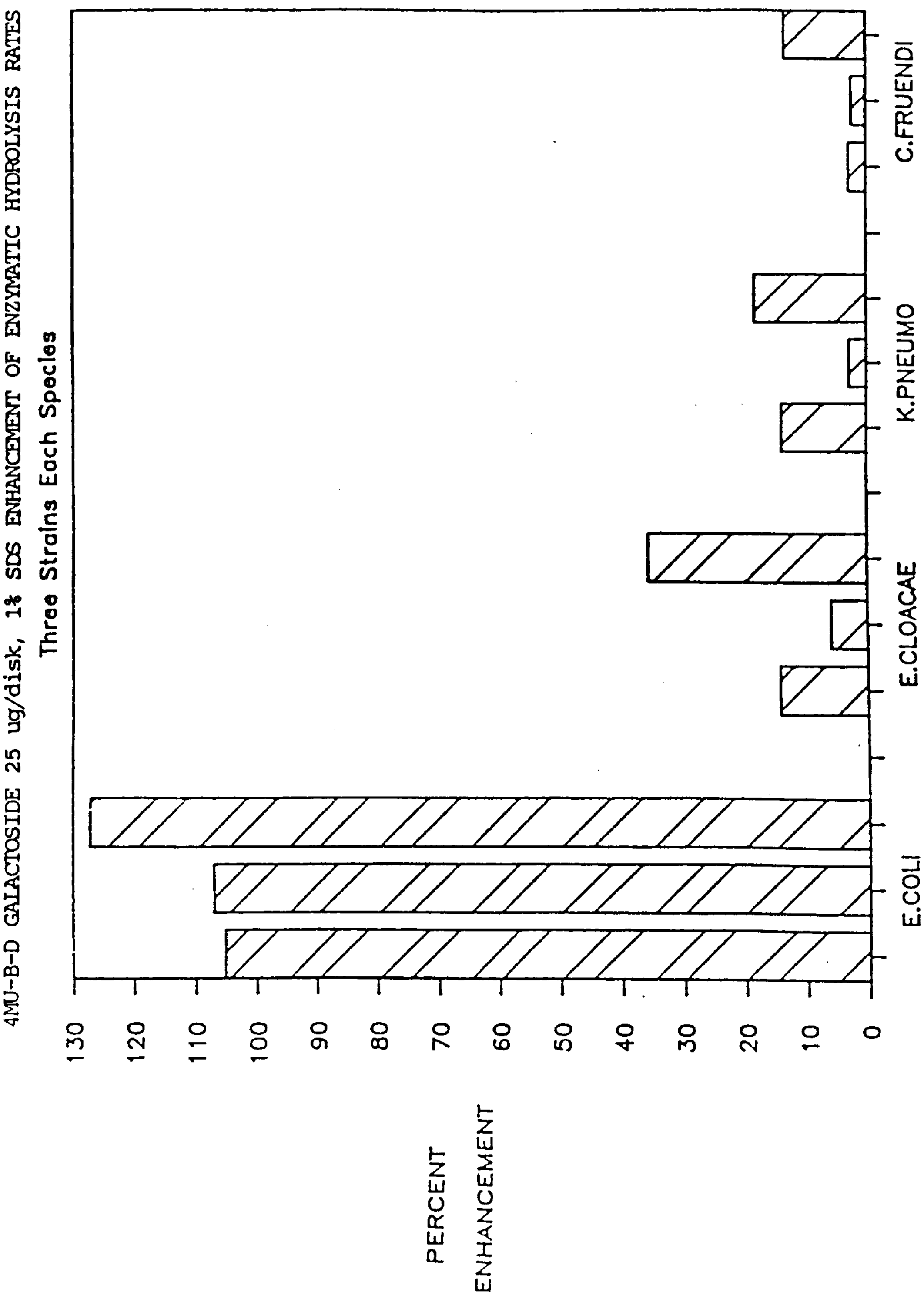
4MU-B-D GALACTOSIDE 25 ug/disk, 1% SDS ENHANCEMENT OF ENZYMATIC HYDROLYSIS RATES
Three Strains Each Species
PERCENT
ENHANCEMENT
130
120
110
100
90
80
70
60
50
40
30
20
10
0
E.COLI
E.CLOACAE
K.PNEUMO
C.FRUENDI
ORGANISM

FIG.5

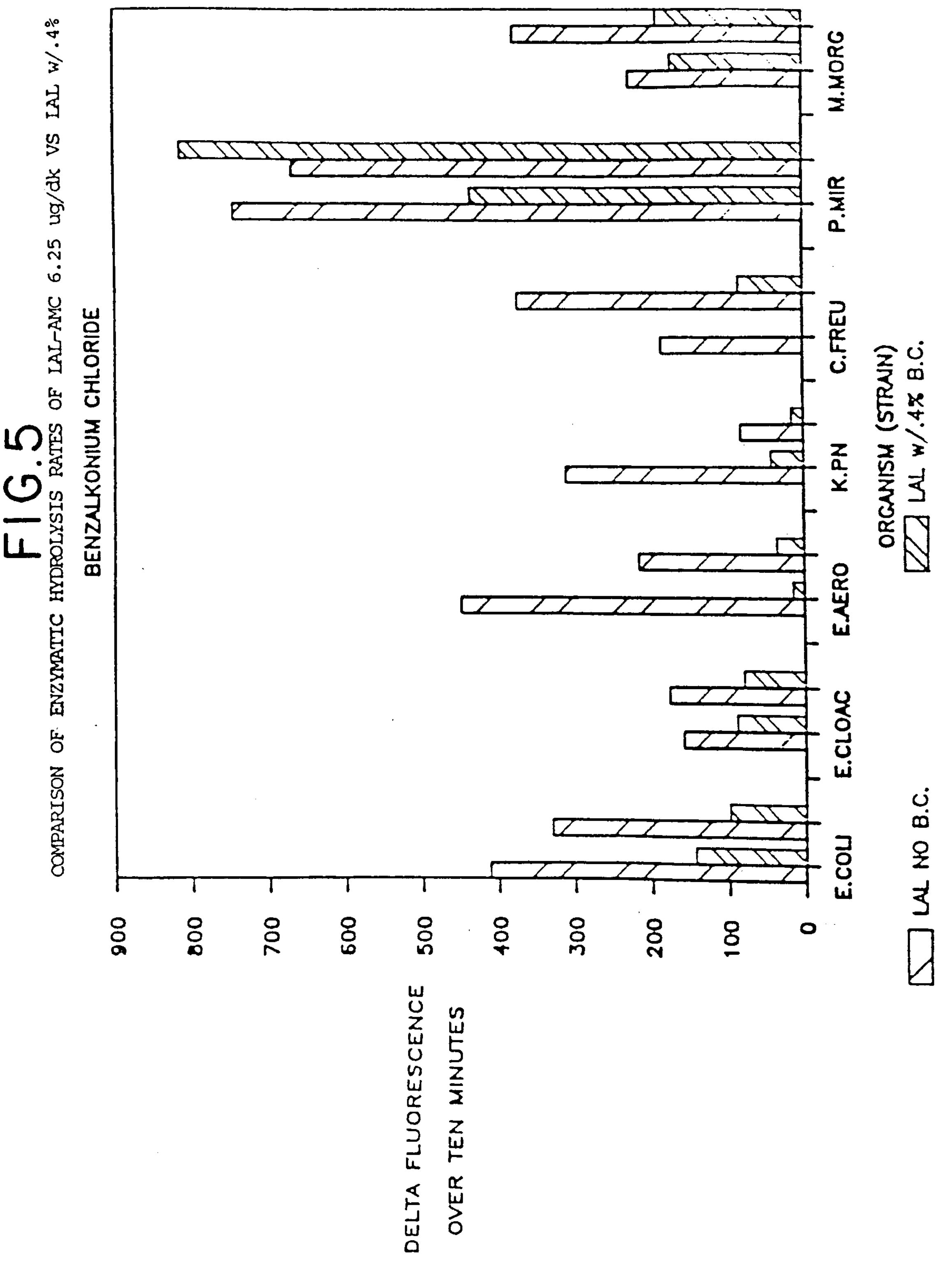
COMPARISON OF ENZYMATIC HYDROLYSIS RATES OF LAL-AMC 6.25 ug/dk VS LAL w/.4%
BENZALKONIUM CHLORIDE
DELTA FLUORESCENCE
OVER TEN MINUTES
900
800
700
600
500
400
300
200
100
0
E.COLI
E.CLOAC
E.AERO
K.PN
C.FREU
P.MIR
M.MORG
ORGANISM (STRAIN)
LAL NO B.C.
LAL w/.4% B.C.

FIG.6

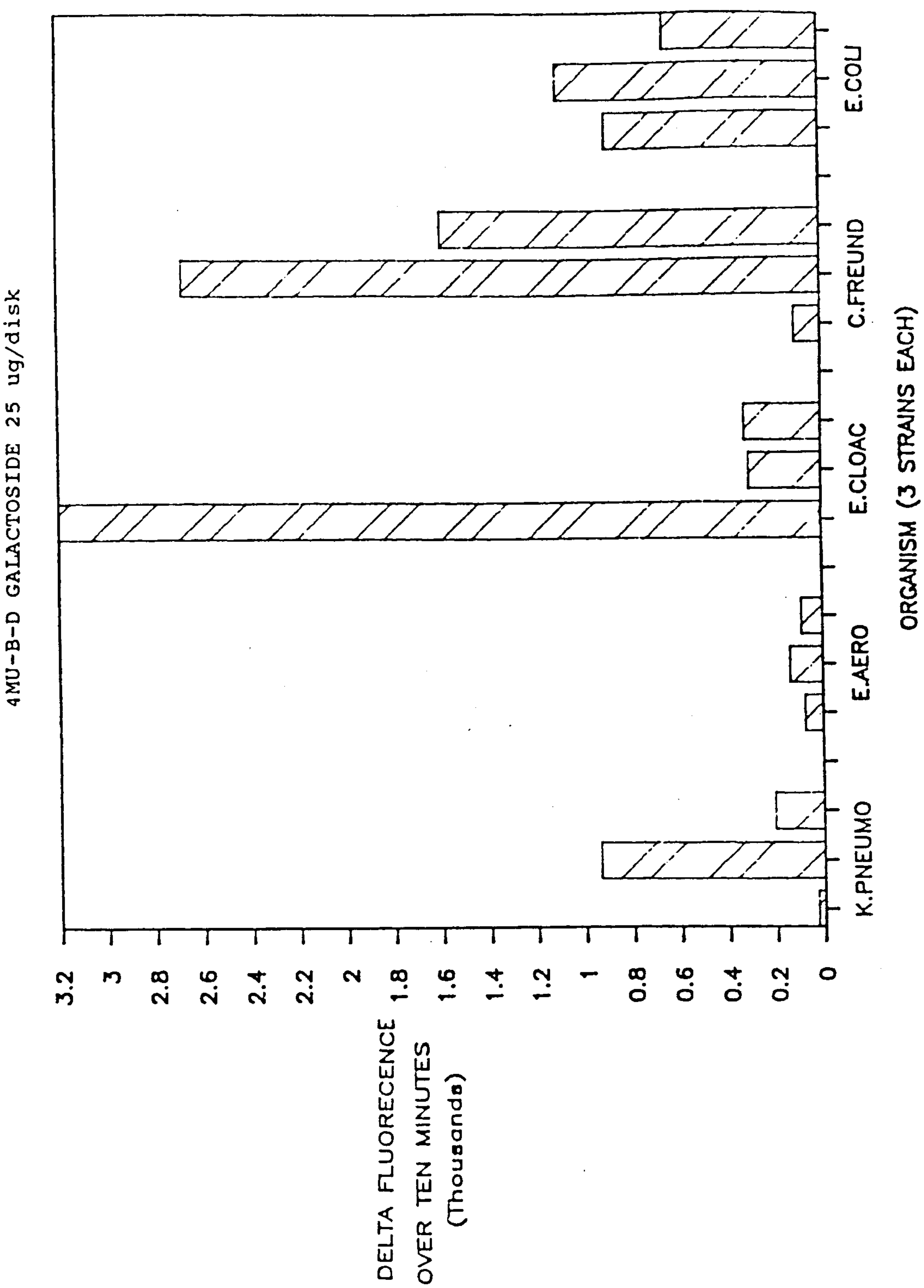
4MU-B-D GALACTOSIDE 25 ug/disk
DELTA FLUORECENCE OVER TEN MINUTES (Thousands)
3.2
3
2.8
2.6
2.4
2.2
2
1.8
1.6
1.4
1.2
1
0.8
0.6
0.4
0.2
0
K.PNEUMO
E.AERO
E.CLOAC
C.FREUND
E.COLI
ORGANISM (3 STRAINS EACH)

FIG.7

4MU-B-D GALACTOSIDE 25 ug/disk

with BENZALKONIUM CHLORIDE, 1.6%

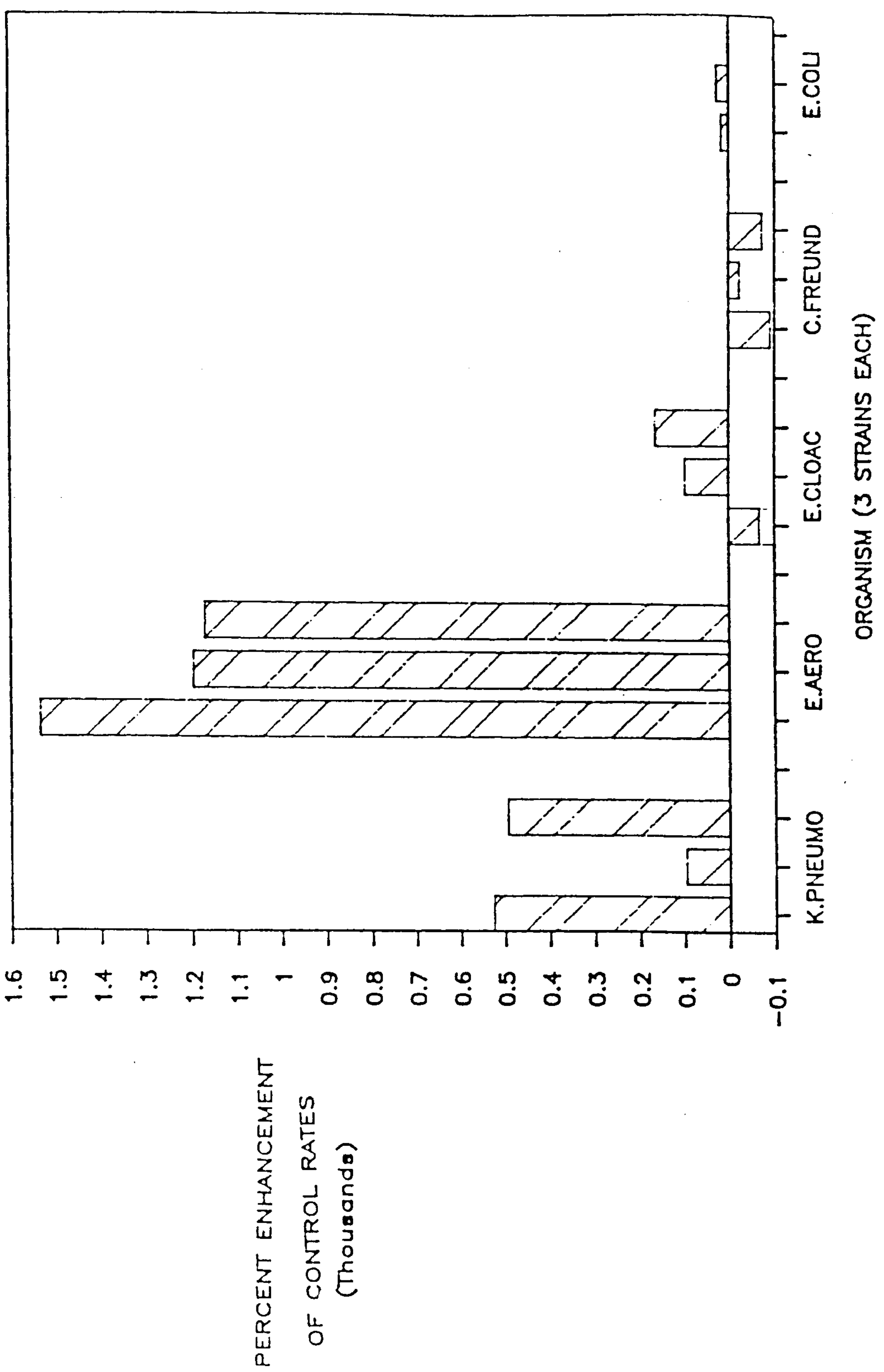

FIG.8

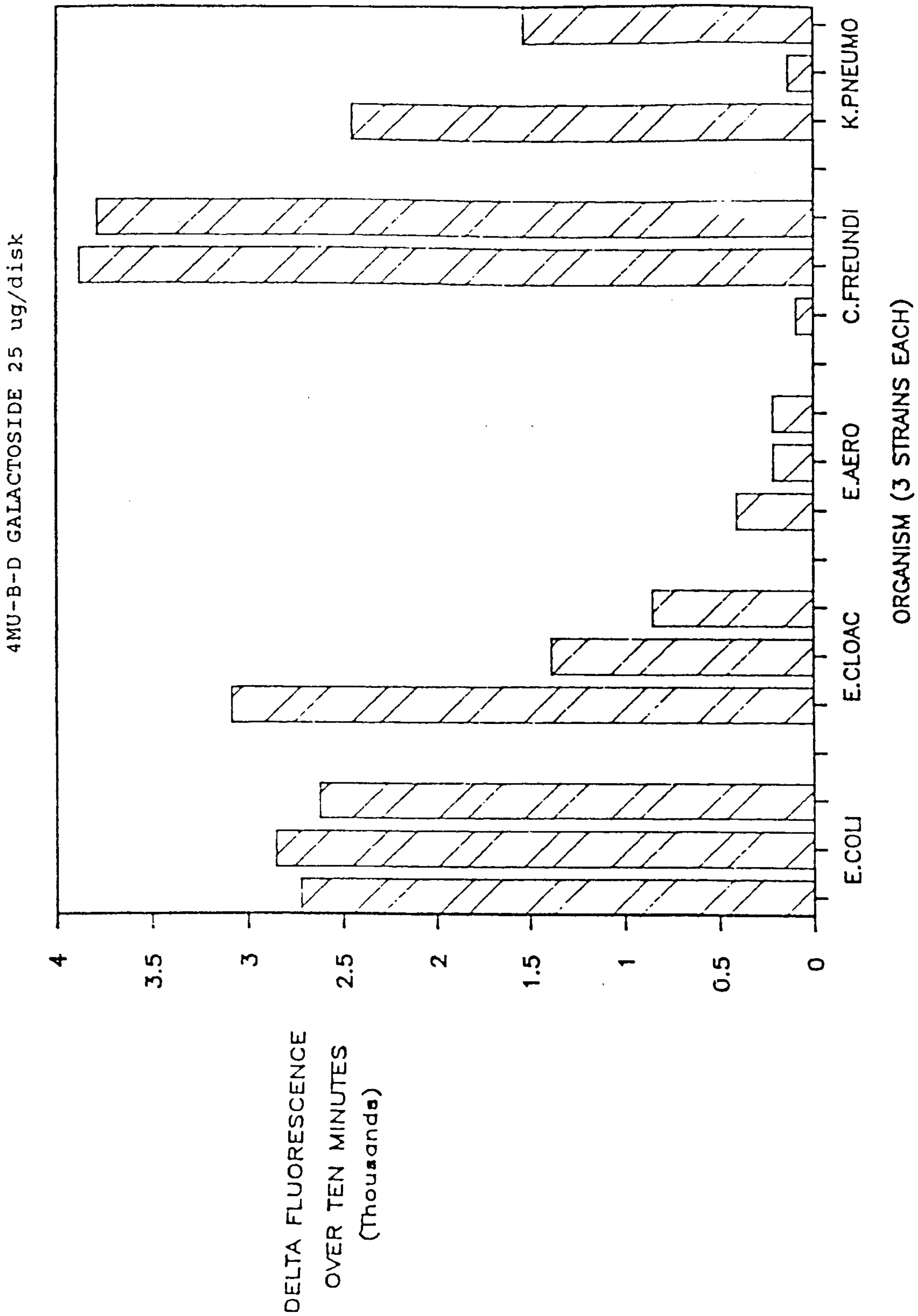
4MU-B-D GALACTOSIDE 25 ug/disk
DELTA FLUORESCENCE
OVER TEN MINUTES
(Thousands)
0
0.5
1
1.5
2
2.5
3
3.5
4
E.COLI
E.CLOAC
E.AERO
C.FREUNDI
K.PNEUMO
ORGANISM (3 STRAINS EACH)

FIG.9

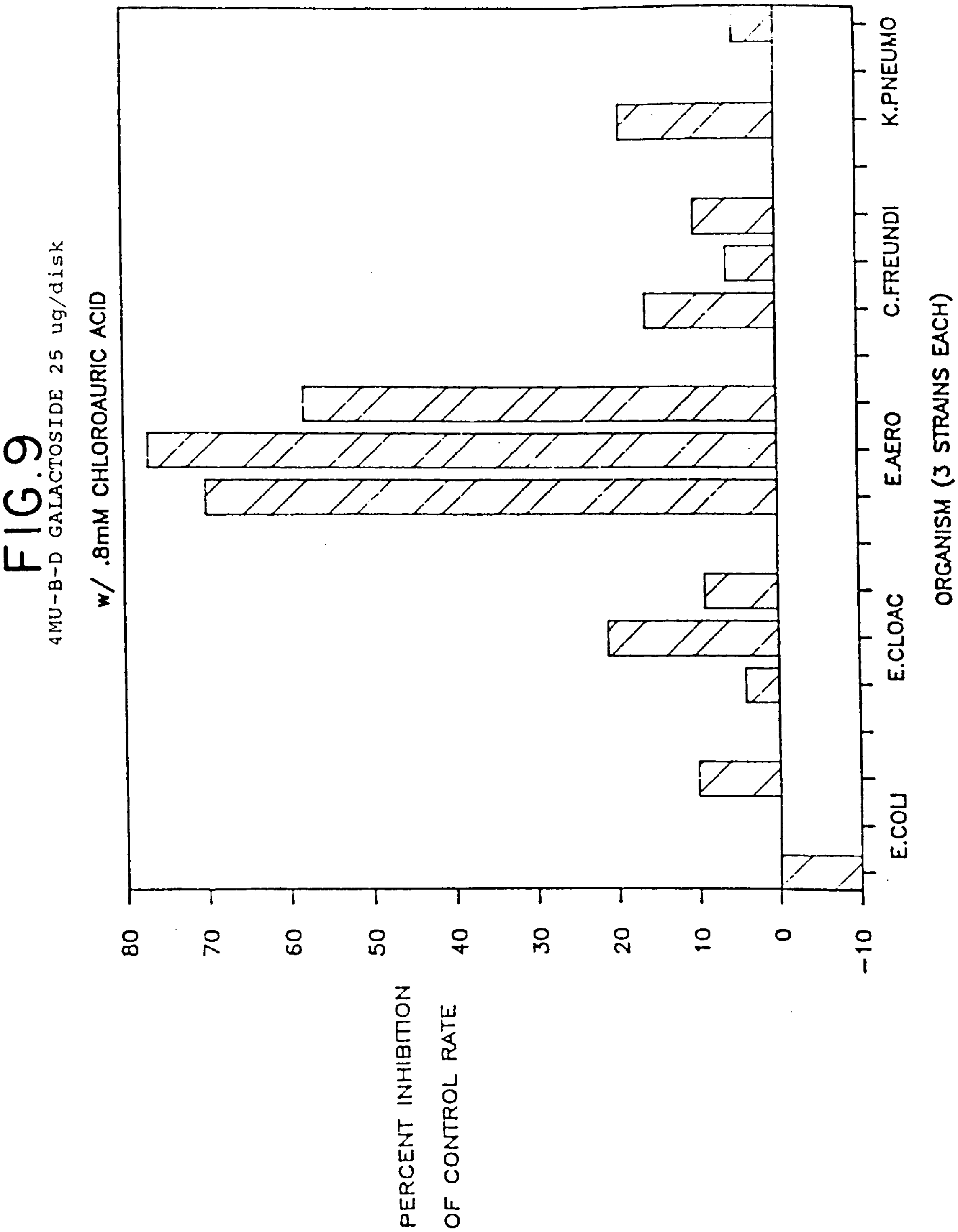
4MU-B-D GALACTOSIDE 25 ug/disk
w/ .8mM CHLOROAURIC ACID
PERCENT INHIBITION
OF CONTROL RATE
80
70
60
50
40
30
20
10
0
-10
E.COLI
E.CLOAC
E.AERO
C.FREUNDI
K.PNEUMO
ORGANISM (3 STRAINS EACH)

FIG.10

L-ALANINE-AMC, 6.25 ug/disk

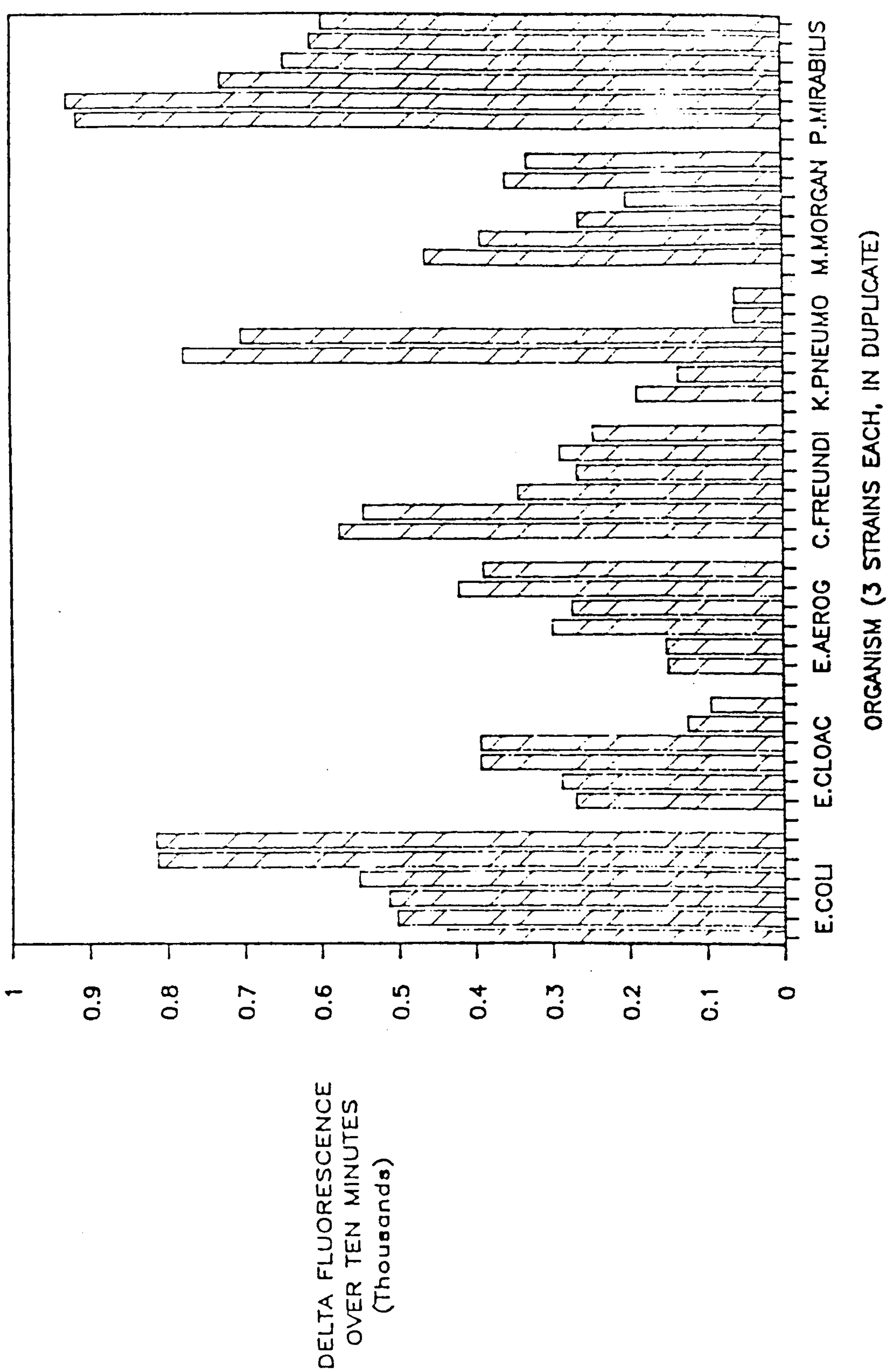

FIG. 11

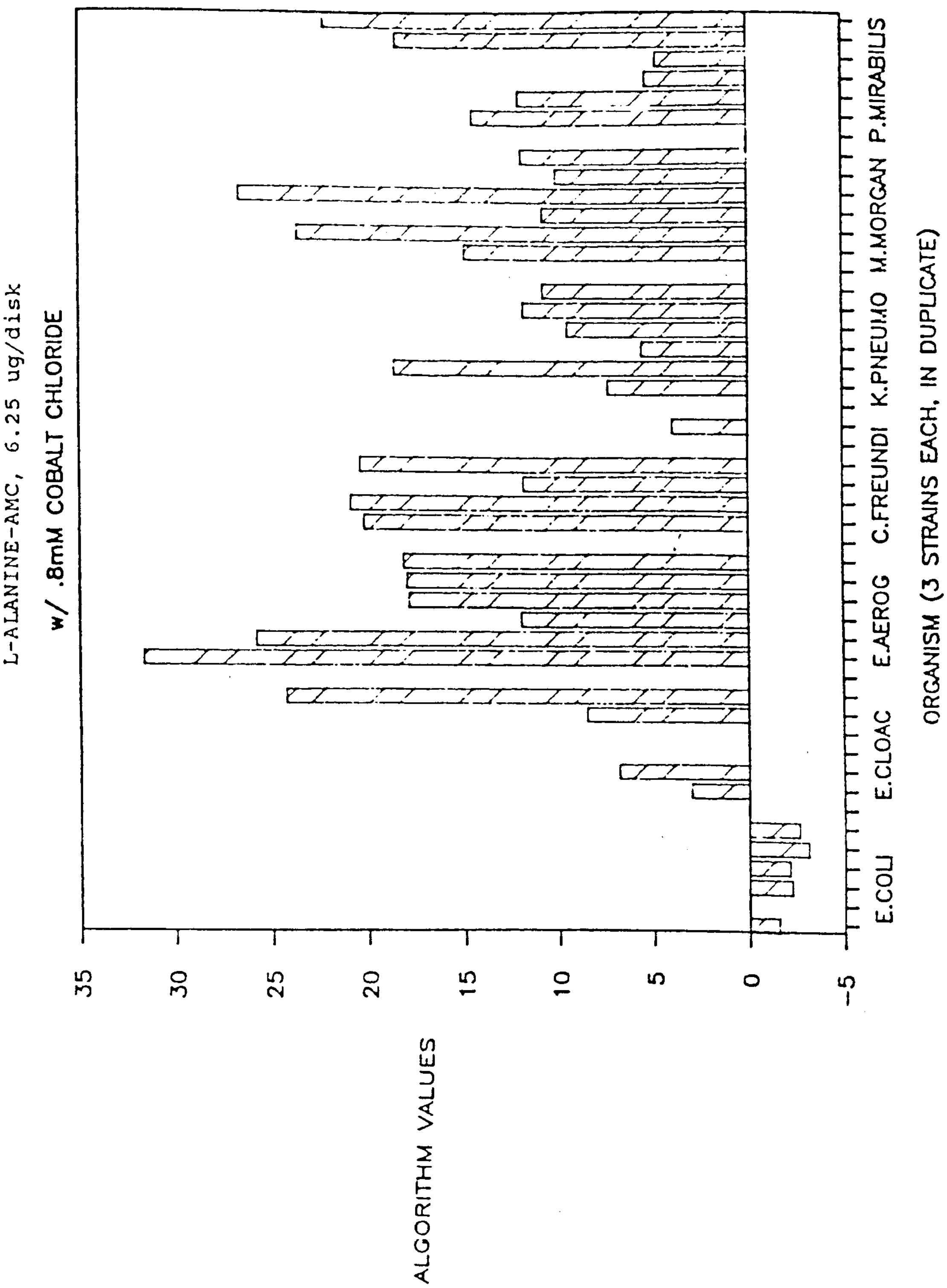
L-ALANINE-AMC, 6.25 ug/disk
w/ .8mM COBALT CHLORIDE
ALGORITHM VALUES
35
30
25
20
15
10
5
0
-5
E.COLI
E.CLOAC
E.AEROG
C.FREUNDI
K.PNEUMO
M.MORGAN
P.MIRABILIS
ORGANISM (3 STRAINS EACH, IN DUPLICATE)

FIG.12

L-ALANINE-AMC, 6.25 ug/disk

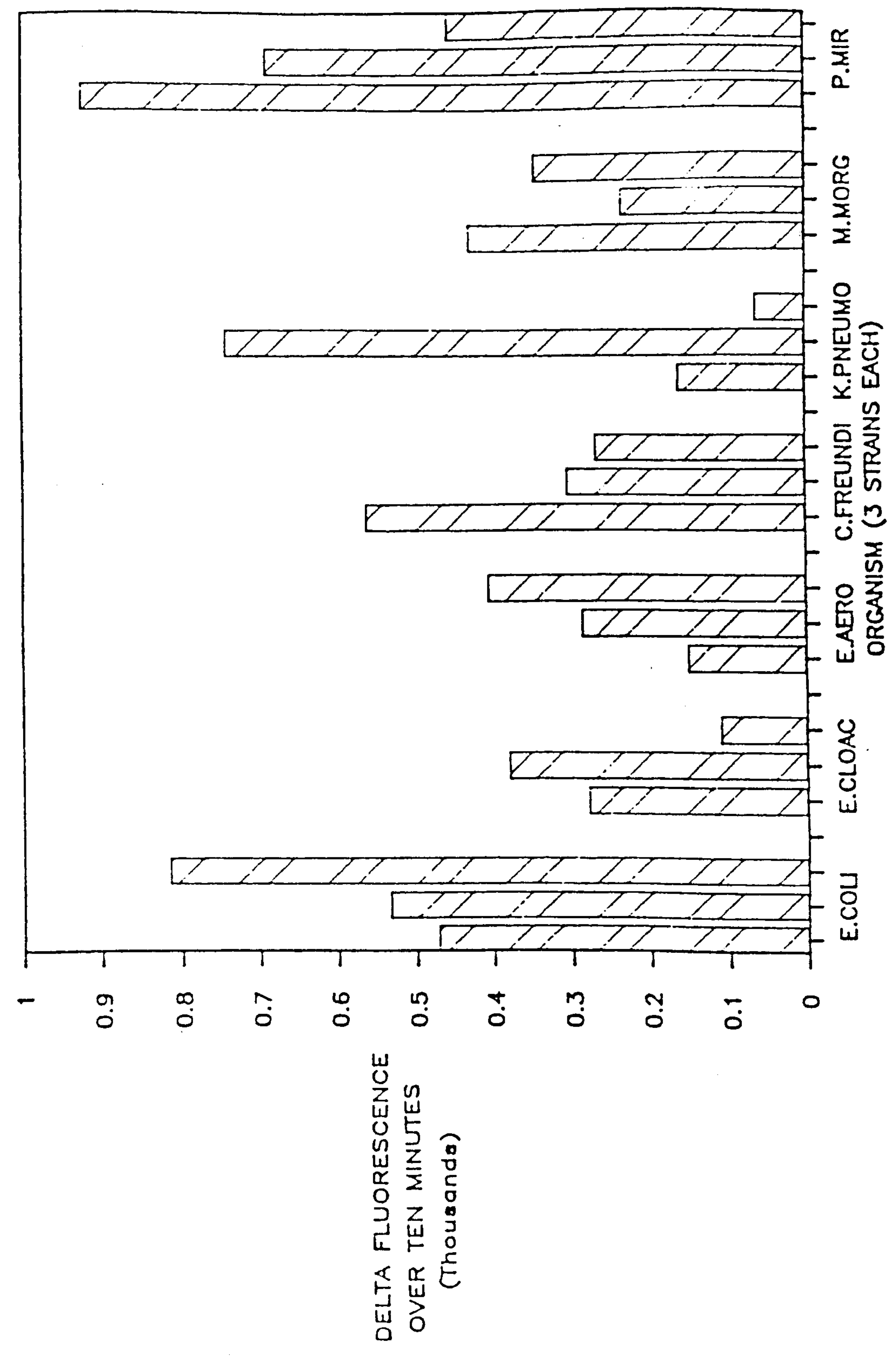

FIG.13

L-ALANINE-AMC, 6.25 ug/disk
w/ 8mM ZINC CHLORIDE

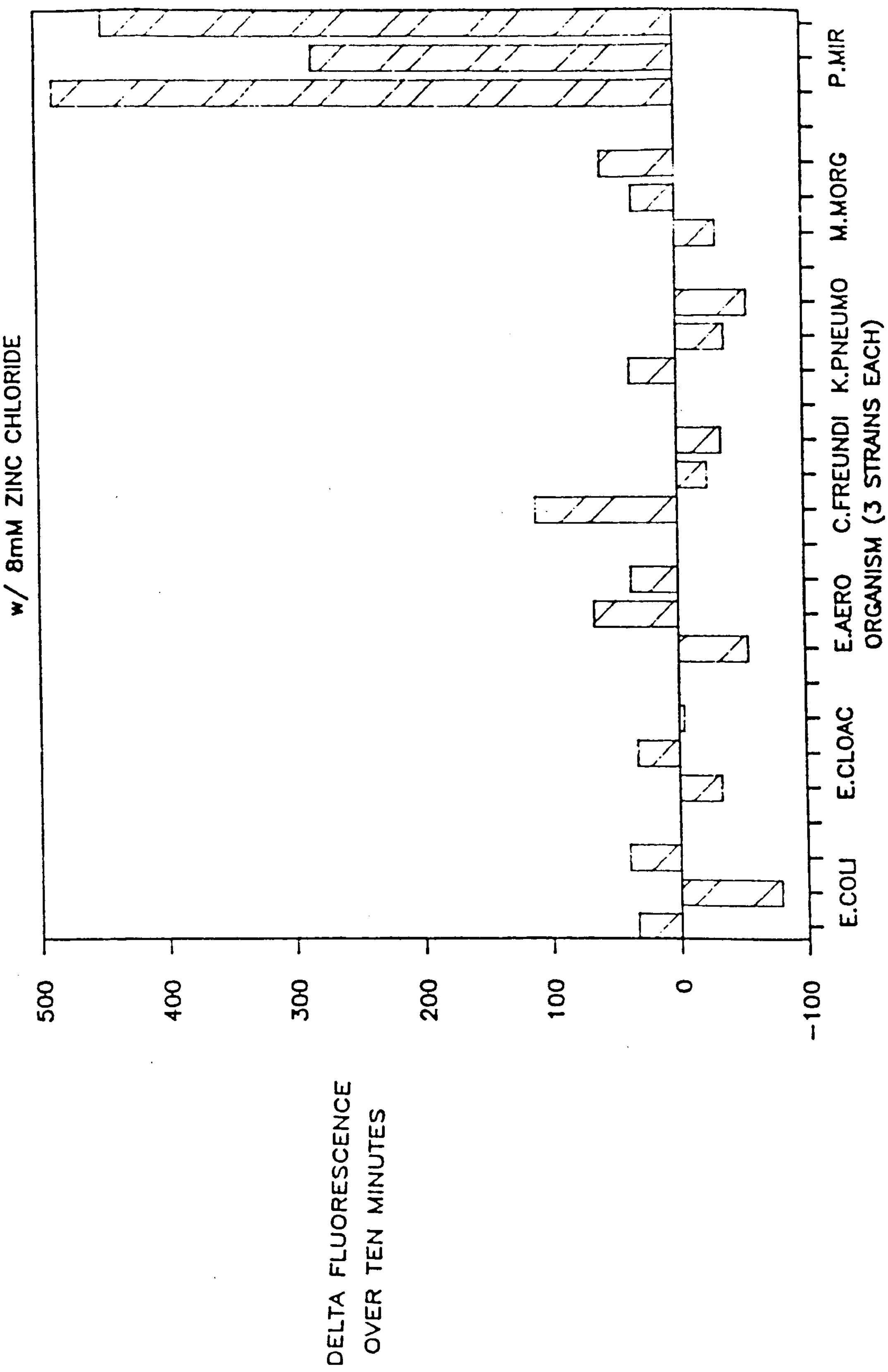

FIG.14

4MU-B-D GLUCURONIDE 25 ug/disk

NO ACTIVITY AFFECTING AGENTS ADDED

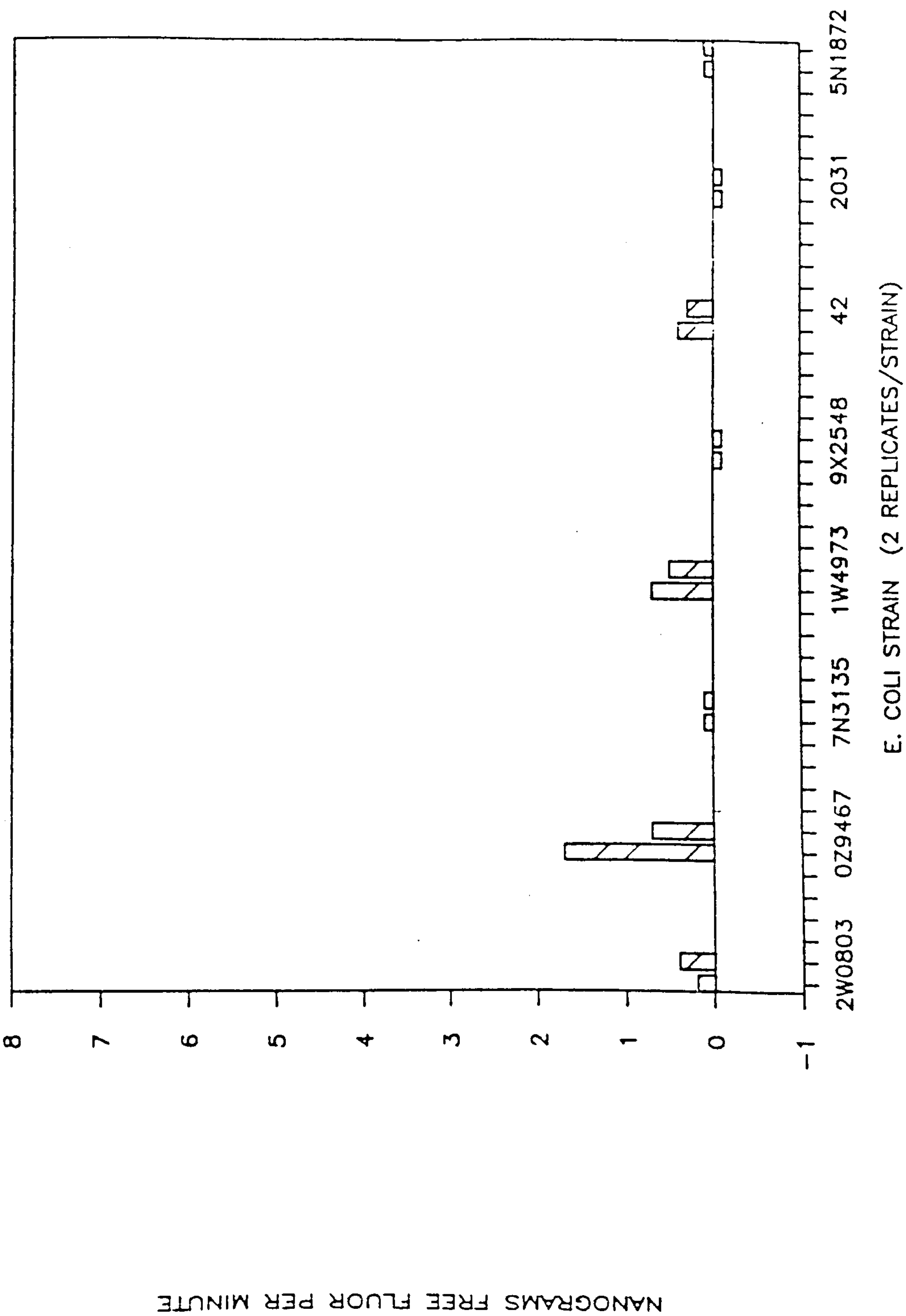

FIG.15

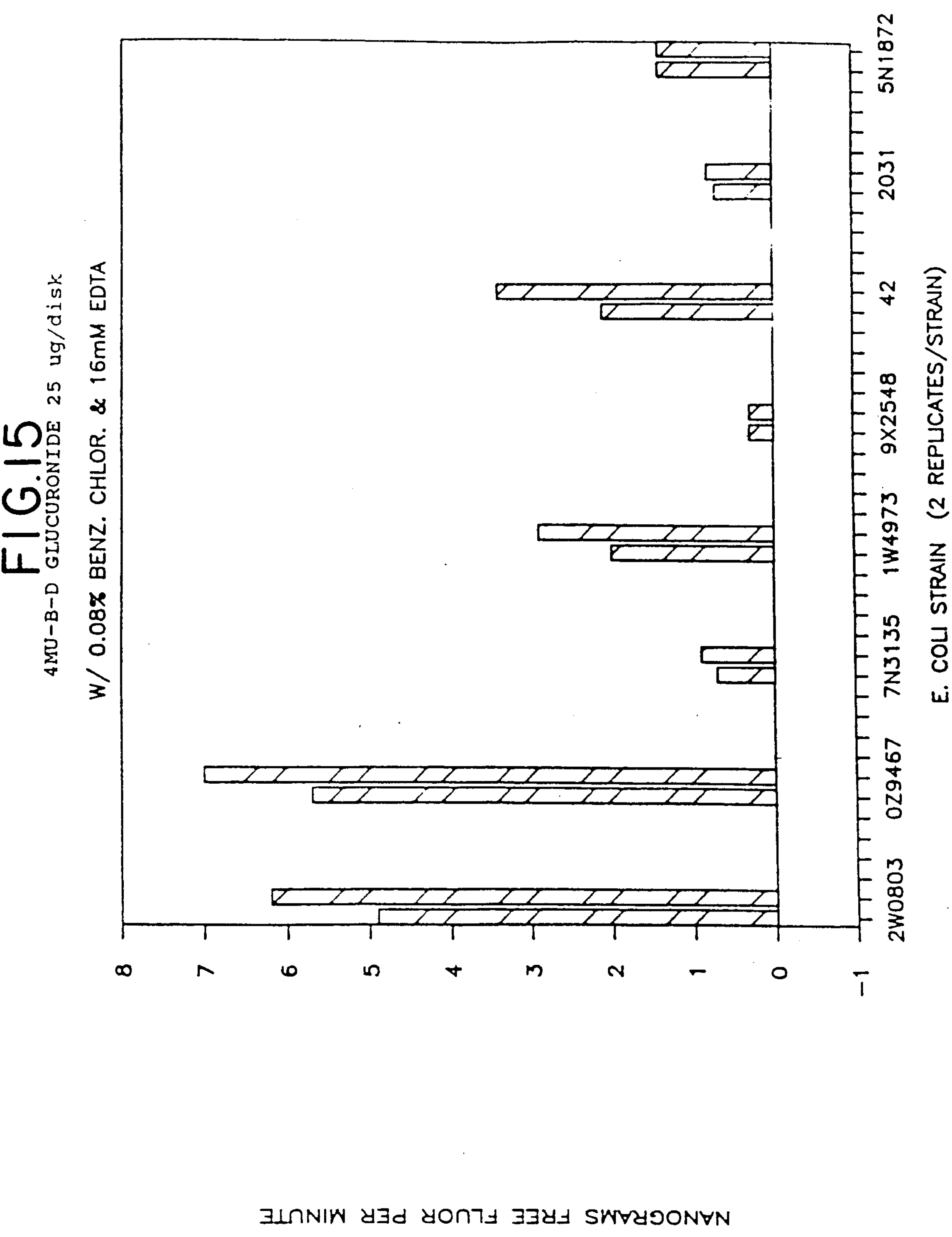
4MU-B-D GLUCURONIDE 25 ug/disk
W/ 0.08% BENZ. CHLOR. & 16mM EDTA
NANOGRAMS FREE FLUOR PER MINUTE
8
7
6
5
4
3
2
1
0
−1
2W0803
0Z9467
7N3135
1W4973
9X2548
42
2031
5N1872
E. COLI STRAIN (2 REPLICATES/STRAIN)

# FIG.16

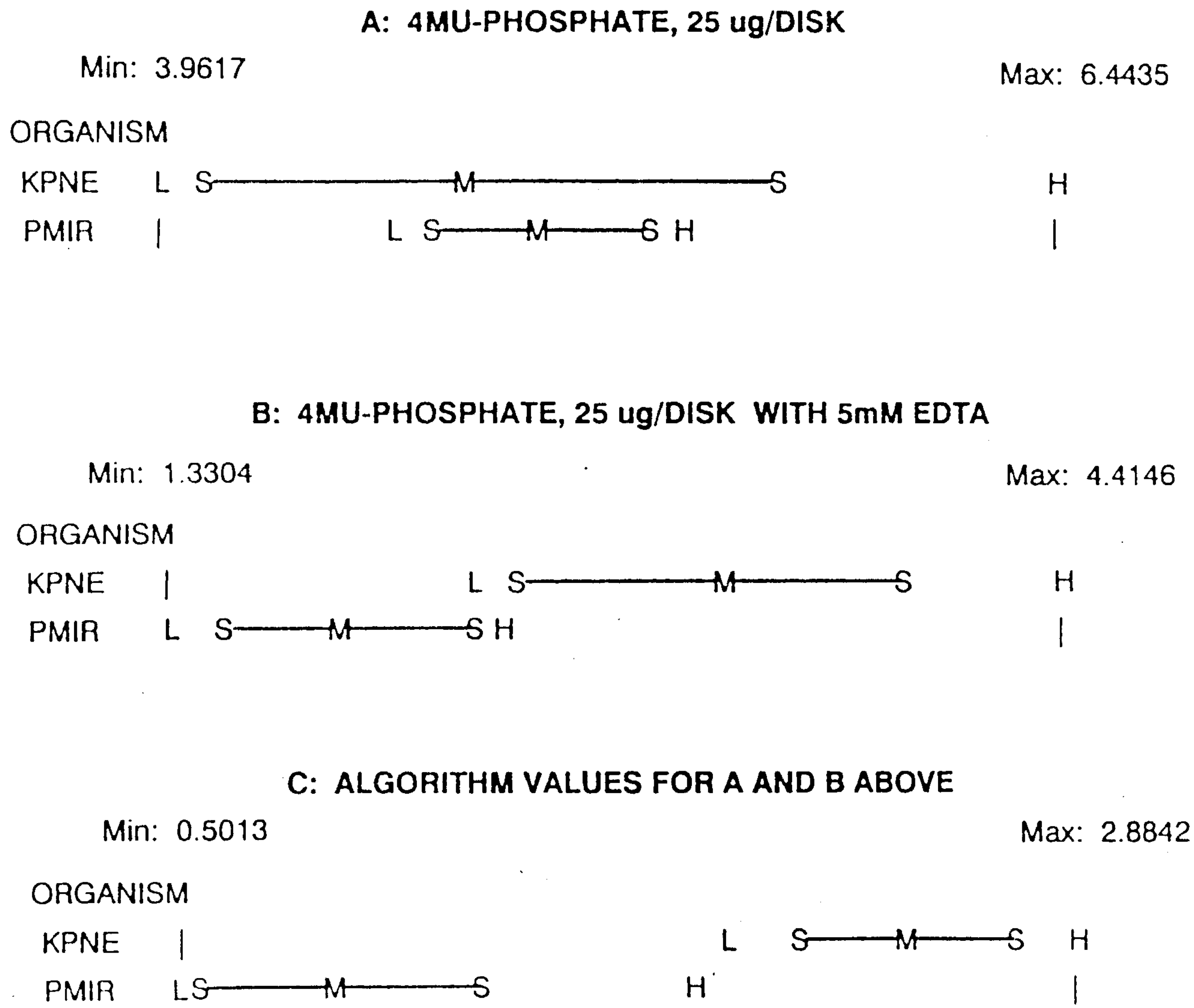
A: 4MU-PHOSPHATE, 25 ug/DISK
Min: 3.9617
Max: 6.4435
ORGANISM
KPNE
PMIR
L S M S H
L S M S H
B: 4MU-PHOSPHATE, 25 ug/DISK WITH 5mM EDTA
Min: 1.3304
Max: 4.4146
ORGANISM
KPNE
PMIR
L S M S H
L S M S H
C: ALGORITHM VALUES FOR A AND B ABOVE
Min: 0.5013
Max: 2.8842
ORGANISM
KPNE
PMIR
L S M S H
LS M S H

FIG.17

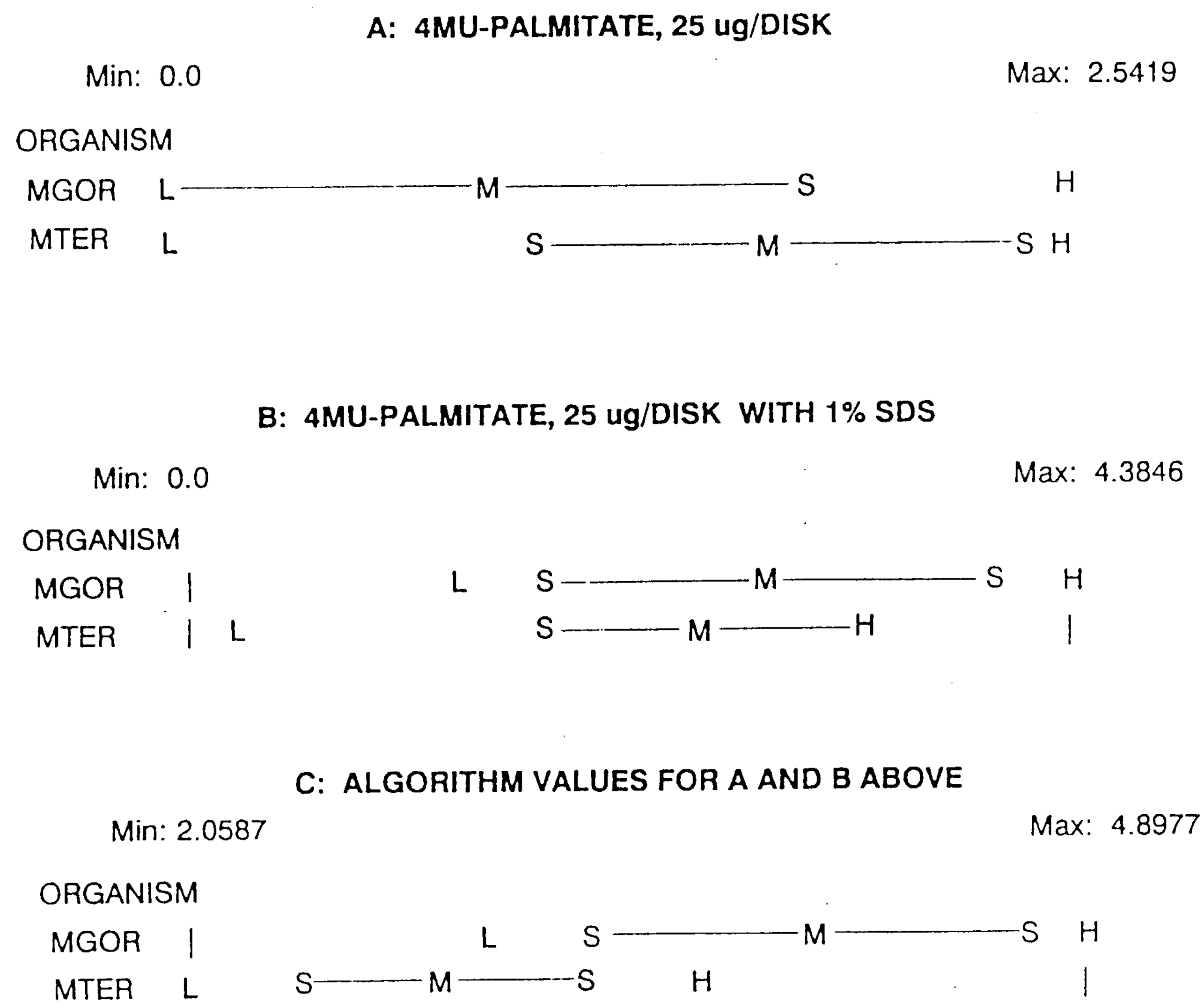
A: 4MU-PALMITATE, 25 ug/DISK
Min: 0.0
Max: 2.5419
ORGANISM
MGOR L M S H
MTER L S M S H
B: 4MU-PALMITATE, 25 ug/DISK WITH 1% SDS
Min: 0.0
Max: 4.3846
ORGANISM
MGOR L S M S H
MTER L S M H
C: ALGORITHM VALUES FOR A AND B ABOVE
Min: 2.0587
Max: 4.8977
ORGANISM
MGOR L S M S H
MTER L S M S H